# EUROPEAN PATENT APPLICATION

(11) **EP 4 494 964 A1**
(43) Date of publication of application: **22.01.2025**
(21) Application number: 24190046.3
(22) Date of filing: 22.07.2024
(51) Int. Cl.: B60W 40/09, B60W 50/02, B60W 60/00, G09B 9/04, G09B 9/042, G09B 9/052, B60W 50/14

(54) **APPARATUS AND METHOD FOR SAFETY DRIVER TRAINING**

(30) Priority: 21.07.2023 US 202363514926 P
(71) Applicant: Tusimple, Inc., San Diego, CA 92122 (US)
(72) Inventor: PANTTILA, John E., Tuscon (US); LI, Dishi, Menifee (US); CHO, HongSeok, Foster City (US); HSU, Yu-Ju, Tuscon (US); ZHANG, Cheng, San Diego (US)
(74) Representative: Morrall, Jonathan Ian McLachlan

(57) **Abstract**

A computer-implemented method and apparatus are provided for training a safety driver associated with an autonomous vehicle (AV) operating in an autonomous driving mode. The computer-implemented method includes receiving (902) a selection of one or more vehicle operation faults that are configured to impact operation of one or more vehicle systems of the AV. The computer-implemented method also includes injecting (904) the one or more vehicle operation faults into one or more vehicle systems of the AV and detecting (906) one or more driver responses executed by the safety driver in response to the one or more vehicle operation faults. The computer-implemented method further includes determining (908) a reaction time associated with the one or more driver responses. The reaction time is a measure of time it takes the safety driver to respond to the one or more vehicle operation faults.

## Description

### TECHNICAL FIELD

The present disclosure relates generally to autonomous vehicles (AVs). More specifically, the present disclosure is related to an AV safety driver training system configured to inject one or more vehicle operation faults into an AV operating in an autonomous driving mode in order to train an AV safety driver.

### BACKGROUND

One aim of autonomous vehicle technologies is to provide vehicles that can safely navigate toward a destination with limited or no driver assistance. The effective navigation of an AV from one location to another may include the ability to signal other vehicles, navigate around other vehicles in shoulders or emergency lanes, change lanes, bias appropriately in a lane, and navigate all portions or types of highway lanes. Autonomous vehicle technologies may enable an AV to operate without requiring extensive learning, training or unconventional behavior by nearby drivers proximate the AV, by ensuring that the AV can operate effectively, in a way that is evident, logical, or familiar to nearby drivers and pedestrians.

In order for an enterprise to operate a fleet of AVs in an autonomous vehicle network, the AVs must operate effectively amongst other vehicles that are traveling on public roadways. Furthermore, any AV operating in an autonomous vehicle network by way of public roadways must comply with various safety standards and regulations including federal, state and/or local safety standards and regulations (hereinafter collectively and individually referenced as safety standards and regulations). Moreover, in many circumstances, a safety driver is required to be onboard an AV while the AV is operating in an autonomous driving mode in order to mitigate any potentially adverse events caused by a malfunctioning vehicle system of the AV. As such, a safety driver may be trained to take effective, safe control of an AV that is operating on public roadways.

### BRIEF SUMMARY

In an example embodiment, an apparatus is provided that includes at least one processor and at least one non-transitory memory including computer program code instructions. The computer program code instructions are configured to, when executed, cause the apparatus to receive a selection of one or more vehicle operation faults from a plurality of vehicle operation faults. The plurality of vehicle operation faults is configured to impact an operation of one or more vehicle systems of an autonomous vehicle operating in an autonomous driving mode. The apparatus is also caused to inject the one or more vehicle operation faults to the one or more vehicle systems of the autonomous vehicle and to detect one or more driver responses executed by a safety driver in response to the one or more vehicle operation faults. The apparatus is further caused to determine a reaction time associated with the one or more driver responses detected to be executed by the safety driver. The reaction time is a measure of time it takes the safety driver to respond to the one or more vehicle operation faults injected into the one or more vehicle systems of the autonomous vehicle.

The plurality of vehicle operation faults may include at least one of one or more steering faults, acceleration faults, deceleration faults, braking faults, stopping procedure faults, or vehicle trailer faults. Each vehicle operation fault of the plurality of vehicle operation faults may be associated with a predefined fault injection duration. In an example embodiment, the computer program code instructions configured to detect the one or more driver responses executed by the safety driver further cause the apparatus to determine one or more driver input values associated with the one or more respective driver responses to the one or more vehicle operation faults with the one or more driver input values being associated with one or more respective controls associated with the one or more vehicle systems of the autonomous vehicle. The one or more driver responses may include at least one of a steering response, a braking response, an acceleration response, a deceleration response, or a gear shifting response. At least one of the one or more driver responses may cause the autonomous vehicle to disengage from the autonomous driving mode and switch to a manual driving mode.

The computer program code instructions of an example embodiment further cause the apparatus to generate, in response to injecting the one or more vehicle operation faults, one or more fault event tags. The one or more fault event tags include one or more portions of data related to at least one of a fault event tag identifier, vehicle operation fault injection timestamp data, the one or more vehicle operation faults, the one or more driver responses executed by the safety driver in response to the one or more vehicle operation faults, one or more reaction times associated with the safety driver, one or more driver input values, or one or more autonomous driving mode disengagement methods. In an example embodiment, the computer program code instructions further cause the apparatus to generate an AV safety driver training session report. The AV safety driver training session report includes at least one of one or more fault event tags, one or more portions of image data associated with the safety driver, one or more portions of video data associated with the safety driver, or one or more portions of audio data associated with the safety driver, and wherein the safety driver training session report is associated with a respective AV safety driver training session of the safety driver.

In another example embodiment, a computer-implemented method is provided that includes receiving a selection of one or more vehicle operation faults from a plurality of vehicle operation faults. The plurality of vehicle operation faults is configured to impact an operation of one or more vehicle systems of an autonomous vehicle operating in an autonomous driving mode. The computer-implemented method also includes injecting the one or more vehicle operation faults to the one or more vehicle systems of the autonomous vehicle and detecting one or more driver responses executed by a safety driver in response to the one or more vehicle operation faults. The computer-implemented method further includes determining a reaction time associated with the one or more driver responses detected to be executed by the safety driver. The reaction time is a measure of time it takes the safety driver to respond to the one or more vehicle operation faults injected into the one or more vehicle systems of the autonomous vehicle.

The plurality of vehicle operation faults may include at least one of one or more steering faults, acceleration faults, deceleration faults, braking faults, stopping procedure faults, or vehicle trailer faults. Each vehicle operation fault of the plurality of vehicle operation faults may be associated with a predefined fault injection duration. In an example embodiment, detecting the one or more driver responses executed by the safety driver includes determining one or more driver input values associated with the one or more respective driver responses to the one or more vehicle operation faults with the one or more driver input values being associated with one or more respective controls associated with the one or more vehicle systems of the autonomous vehicle. The one or more driver responses may include at least one of a steering response, a braking response, an acceleration response, a deceleration response, or a gear shifting response. At least one of the one or more driver responses may cause the autonomous vehicle to disengage from the autonomous driving mode and switch to a manual driving mode.

The computer-implemented method of an example embodiment may also include generating, in response to injecting the one or more vehicle operation faults, one or more fault event tags. The one or more fault event tags include one or more portions of data related to at least one of a fault event tag identifier, vehicle operation fault injection timestamp data, the one or more vehicle operation faults, the one or more driver responses executed by the safety driver in response to the one or more vehicle operation faults, one or more reaction times associated with the safety driver, one or more driver input values, or one or more autonomous driving mode disengagement methods. In an example embodiment, the computer-implemented method also includes generating an AV safety driver training session report. The AV safety driver training session report includes at least one of one or more fault event tags, one or more portions of image data associated with the safety driver, one or more portions of video data associated with the safety driver, or one or more portions of audio data associated with the safety driver. The safety driver training session report is associated with a respective AV safety driver training session of the safety driver.

In a further example embodiment, a computer program product is provided that includes at least one non-transitory computer-readable storage medium having computer-executable program code instructions stored therein. The computer-executable program code instructions include program code instructions configured to receive a selection of one or more vehicle operation faults from a plurality of vehicle operation faults. The plurality of vehicle operation faults is configured to impact an operation of one or more vehicle systems of an autonomous vehicle operating in an autonomous driving mode. The computer-executable program code instructions also include program code instructions configured to inject the one or more vehicle operation faults to the one or more vehicle systems of the autonomous vehicle and program code instructions configured to detect one or more driver responses executed by a safety driver in response to the one or more vehicle operation faults. The computer-executable program code instructions further include program code instructions configured to determine a reaction time associated with the one or more driver responses detected to be executed by the safety driver, wherein the reaction time is a measure of time it takes the safety driver to respond to the one or more vehicle operation faults injected into the one or more vehicle systems of the autonomous vehicle.

The plurality of vehicle operation faults may include at least one of one or more steering faults, acceleration faults, deceleration faults, braking faults, stopping procedure faults, or vehicle trailer faults. Each vehicle operation fault of the plurality of vehicle operation faults may be associated with a predefined fault injection duration. In an example embodiment, the program code instructions configured to detect the one or more driver responses executed by the safety driver include program code instructions configured to determine one or more driver input values associated with the one or more respective driver responses to the one or more vehicle operation faults. The one or more driver input values are associated with one or more respective controls associated with the one or more vehicle systems of the autonomous vehicle. The one or more driver responses may include at least one of a steering response, a braking response, an acceleration response, a deceleration response, or a gear shifting response. At least one of the one or more driver responses may cause the autonomous vehicle to disengage from the autonomous driving mode and switch to a manual driving mode.

The computer-executable program code instructions of an example embodiment also include program code instructions configured to generate, in response to injecting the one or more vehicle operation faults, one or more fault event tags. The one or more fault event tags include one or more portions of data related to at least one of a fault event tag identifier, vehicle operation fault injection timestamp data, the one or more vehicle operation faults, the one or more driver responses executed by the safety driver in response to the one or more vehicle operation faults, one or more reaction times associated with the safety driver, one or more driver input values, or one or more autonomous driving mode disengagement methods. In an example embodiment, the computer-executable program code instructions further include program code instructions configured to generate an AV safety driver training session report. The AV safety driver training session report includes at least one of one or more fault event tags, one or more portions of image data associated with the safety driver, one or more portions of video data associated with the safety driver, or one or more portions of audio data associated with the safety driver. The safety driver training session report is associated with a respective AV safety driver training session of the safety driver.

In yet another example embodiment, an apparatus is provided that includes means for receiving a selection of one or more vehicle operation faults from a plurality of vehicle operation faults. The plurality of vehicle operation faults is configured to impact an operation of one or more vehicle systems of an autonomous vehicle operating in an autonomous driving mode. The apparatus also includes means for injecting the one or more vehicle operation faults to the one or more vehicle systems of the autonomous vehicle and means for detecting one or more driver responses executed by a safety driver in response to the one or more vehicle operation faults. The apparatus further includes means for determining a reaction time associated with the one or more driver responses detected to be executed by the safety driver. The reaction time is a measure of time it takes the safety driver to respond to the one or more vehicle operation faults injected into the one or more vehicle systems of the autonomous vehicle.

The plurality of vehicle operation faults may include at least one of one or more steering faults, acceleration faults, deceleration faults, braking faults, stopping procedure faults, or vehicle trailer faults. Each vehicle operation fault of the plurality of vehicle operation faults may be associated with a predefined fault injection duration. In an example embodiment, the means for detecting the one or more driver responses executed by the safety driver includes means for determining one or more driver input values associated with the one or more respective driver responses to the one or more vehicle operation faults with the one or more driver input values being associated with one or more respective controls associated with the one or more vehicle systems of the autonomous vehicle. The one or more driver responses may include at least one of a steering response, a braking response, an acceleration response, a deceleration response, or a gear shifting response. At least one of the one or more driver responses may cause the autonomous vehicle to disengage from the autonomous driving mode and switch to a manual driving mode.

The apparatus of an example embodiment may also include means for generating, in response to injecting the one or more vehicle operation faults, one or more fault event tags. The one or more fault event tags include one or more portions of data related to at least one of a fault event tag identifier, vehicle operation fault injection timestamp data, the one or more vehicle operation faults, the one or more driver responses executed by the safety driver in response to the one or more vehicle operation faults, one or more reaction times associated with the safety driver, one or more driver input values, or one or more autonomous driving mode disengagement methods. In an example embodiment, the apparatus also includes means for generating an AV safety driver training session report. The AV safety driver training session report includes at least one of one or more fault event tags, one or more portions of image data associated with the safety driver, one or more portions of video data associated with the safety driver, or one or more portions of audio data associated with the safety driver. The safety driver training session report is associated with a respective AV safety driver training session of the safety driver.

### BRIEF DESCRIPTION OF THE DRAWINGS

For a more complete understanding of this disclosure, reference is now made to the following brief description, taken in connection with the accompanying drawings and detailed description, wherein like reference numerals represent like parts.
FIG. 1 illustrates a diagram of an example AV configured to implement autonomous driving operations in accordance with some embodiments discussed herein;
FIG. 2 illustrates a block diagram of a control subsystem in accordance with some embodiments discussed herein;
FIG. 3 illustrates a diagram of an in-vehicle control computer included in an AV in accordance with some embodiments discussed herein;
FIG. 4 illustrates an exemplary system associated with an interactive safety driver training interface for controllably injecting faults, in accordance with some embodiments discussed herein;
FIG. 5 illustrates an exemplary fault injection system in accordance with some embodiments discussed herein;
FIG. 6 illustrates an exemplary user computing device that includes the fault injection system in accordance with some embodiments discussed herein;
FIG. 7 illustrates a system that provides an exemplary environment related to AV safety driver training, in accordance with some embodiments discussed herein;
FIG. 8 is a process flow diagram of an example process for executing a plurality of AV safety driver training operations in accordance with some embodiments discussed herein;
FIG. 9 is a process flow diagram of an example method for executing a plurality of AV safety driver training operations in accordance with some embodiments discussed herein; and
FIG. 10 is an operational example of an interactive safety driver training interface in accordance with some embodiments discussed herein.

### DETAILED DESCRIPTION

Some embodiments of the present disclosure will now be described more fully hereinafter with reference to the accompanying drawings, in which some, but not all embodiments are shown. Indeed, various embodiments of the disclosure may be embodied in many different forms and should not be construed as limited to the embodiments set forth herein. Like reference numerals refer to like elements throughout.

Enterprises, operators, and/or customers associated with AV networks need to ensure the safe execution of the various mission objectives of one or more AVs in an AV fleet that are operating on public roadways. Moreover, the enterprises responsible for the AV fleet need to ensure that when a safety driver is onboard an AV during a particular mission the safety driver will know how to quickly mitigate any unsafe scenarios that arise while the AV is operating in an autonomous driving mode.

Embodiments of the present disclosure provide an AV safety driver training system which can be employed to certify that one or more safety drivers associated with an AV fleet are capable of handling any adverse events related to the AV while the AV is operating in an autonomous driving mode such that the AV can continue to operate safely on public streets and highways. In this regard, the AV safety driver training system may be configured to assist in the training, testing, and development of various AV subsystems associated with an AV. For example, the AV safety driver training system is configured to test and/or develop one or more respective components of various control subsystems, vehicle drive subsystems, vehicle sensor subsystems, vehicle control subsystems, and/or the like associated with an AV. Furthermore, the AV safety driver training system is configured to train one or more safety drivers associated with an AV fleet to correctly mitigate one or more mechanical, technical, and/or operational failures associated with the various subsystems associated with the AVs of the AV fleet.

For an enterprise with a large AV fleet, many safety drivers may need to be trained in a short amount of time. Furthermore, for enterprises dispatching AVs such as autonomous tractor trailers, delivery trucks, multi-axle vehicles, and/or the like, the complexity of the operation of the vehicles, as well as the risks inherent with the operation of such large vehicles, can be limiting factors for an enterprise trying to deploy AV technologies safely.

The AV safety driver training system offers the technical advantage of allowing an enterprise to determine an enterprise-defined AV safety driver training protocol such that a preferred training methodology and/or protocol can be executed. For example, the AV safety driver training system can be used during a safety driver training session to inject one or more vehicle operation faults into a particular AV based on a particular enterprise-defined AV safety driver training protocol. The AV safety driver training protocol may be predefined so as to expose a driver to a variety of different vehicle operation faults and to allow the driver to practice his or her reaction thereto. Although the variety of vehicle operation faults to which the AV safety driving training protocol may be predefined to include all or at least the most common vehicle operation faults, the AV safety driver training protocol may also take into account the response of a driver to certain vehicle operation faults and may provide for one or more vehicle operation faults to be repeated, such as to provide the driver with more experience, particularly in an instance in which the response of the driver to a prior iteration of a vehicle operation fault could be improved or did not satisfy expectations. For example, during a safety driver training session, one or more vehicle operation faults can be injected (e.g., introduced, added, programmed) into one or more elements, components, and/or subsystems (hereinafter generally referenced individually and collectively as a component) of an AV that is operating in an autonomous driving mode. In this regard, the autonomous driving mode may be a fully-autonomous driving mode or a partially-autonomous driving mode. The one or more vehicle operation faults can simulate a worst-case scenario or other predefined scenario that a safety driver may experience while onboard an AV that is operating in an autonomous driving mode. After the one or more vehicle operation faults are injected, the AV safety driving training system can determine one or more driver responses executed by the safety driver that serve to override at least aspects of the autonomous driving mode. A driver response can be characterized by a reaction of the safety driver to the one or more vehicle operation faults. Various parameters that characterize a driver response may be captured including, for example, the manner in which the driver applied the brakes including the force with which the brakes were applied and if the force is consistently applied or varied over time and/or the time required to apply the brakes, the manner in which the driver steered the AV including the extent to which the steering wheel was turned and any variations in the steering over time and/or the time required to initially turn the steering wheel and/or the like. As such, the AV safety driving training system can calculate a reaction time associated with the safety driver, where the reaction time is a measure of time it takes the safety driver to respond to the one or more vehicle operation faults injected into one or more components of the AV 102.

A user computing device associated with a safety driver trainer profile that is associated with the AV safety driver training system can direct, via a communications network, an in-vehicle control computer onboard an AV to inject the one or more vehicle operation faults. In various contexts, the one or more vehicle operation faults can cause one or more components associated with one or more respective AV components to automatically engage, disengage, malfunction, and/or otherwise operate in a manner that is unexpected (e.g., dangerous, off-nominal) while the AV is operating in an autonomous driving mode. For example, the one or more vehicle operation faults can include, but are not limited to, at least one of one or more steering faults, acceleration faults, deceleration faults, braking faults, stopping procedure faults, and/or vehicle trailer faults that cause one or more respective AV components (e.g., a vehicle drive subsystem and/or a vehicle control subsystem) to automatically engage, disengage, malfunction, and/or otherwise operate in a manner that is unexpected.

The one or more vehicle operation faults can be assigned a particular fault intensity value. A fault intensity value is a relative input control value applied to the one or more AV subsystems associated with an AV operating in an autonomous driving mode that indicates the extent of a fault that should be applied. For example, a vehicle operation fault configured as a braking fault may be assigned a fault intensity value of 5%, 25%, 50%, 100%, and/or the like with the percentage defining the portion of a predefined maximum fault intensity that should be applied. In this example, the fault intensity value (e.g., 25%) is a relative input control value applied to the braking system of an AV. For example, in the case of a braking fault injection with a fault intensity value of 25%, the AV safety driver training system would cause the braking system of the AV to automatically apply the brakes of the AV at 25% of the total braking capacity of the AV braking system. Similarly, a vehicle operation fault configured as a steering fault (e.g., a left drift or a right drift) may be assigned a fault intensity of 8°, 20°, 30°, 90°, 120°, 180°, and/or the like with the percentage again defining the portion of a predefined maximum fault intensity that should be applied. In this example, the fault intensity value (e.g., 30°) is a relative input control value applied to the steering system of an AV. For example, in the case of a left drift steering fault injection with a fault intensity value of 30°, the AV safety driver training system would cause the steering system of the AV to automatically turn 30° to the left of the direction in which the AV was previously tracking.

In various embodiments, the AV safety driver training system can receive a selection (e.g., by way of a user computing device) of one or more compound vehicle operation faults. A compound vehicle operation fault is a combination of vehicle operation faults that are presented in concert and that affect two or more AV subsystems simultaneously or sequentially. As a non-limiting example, the AV safety driver training system can inject a compound vehicle operation fault into a component of an AV operating in an autonomous driving mode where the compound vehicle operation fault is configured to cause the AV to simultaneously accelerate (e.g., inject an acceleration fault with a fault intensity value of 50%) and drift to the right (e.g., inject a right-steering fault with a fault intensity value of 20°). Thus, a compound vehicle operation fault may be a combination of vehicle operation faults that affect the operation of one or more components of an AV.

As described herein, the AV safety driver training system can determine one or more driver responses executed by a safety driver operating an AV during a particular safety driver training session. The one or more driver responses can be understood to be one or more reactions of the safety driver to the one or more vehicle operation faults injected into components of the AV. In various contexts, the one or more driver responses can include, but are not limited to, at least one of a steering response, a braking response, an acceleration response, a deceleration response, and/or a gear shifting response. The one or more driver responses can include both the initial response and any variations in the response over time. The AV safety driver training system is configured to interface with the AV (e.g., via an in-vehicle control computer) to determine one or more driver input values associated with the one or more respective driver responses. The one or more driver input values are associated with one or more respective controls associated with the one or more vehicle systems of the AV.

As a non-limiting example, the AV safety driver training system can be configured determine a driver input value associated with one or more steering inputs executed by the safety driver (e.g., a degree value and a directional value associated with the one or more respective steering inputs). As another non-limiting example, the AV safety driver training system can determine a driver input value associated with one or more braking inputs executed by the safety driver (e.g., a braking system capacity percentage value associated with the one or more respective braking inputs).

In various embodiments, the AV safety driver training system will automatically remove (e.g., terminate, cancel) the one or more vehicle operation faults upon detecting a driver response. Furthermore, in various embodiments, the AV safety driver training system will automatically disengage the autonomous driving mode of the AV and switch the AV into a manual driving mode such that the safety driver assumes complete control of the AV after executing the first driver response in reaction to the one or more vehicle operation faults. Furthermore, the AV safety driver training system is configured to measure a reaction time of the safety driver. The reaction time is a measure of the time it takes the safety driver to initially respond (e.g., execute one or more driver responses) to the one or more vehicle operation faults injected into the autonomous vehicle. Moreover, the AV safety driver training system is configured to also measure the effectiveness of the driver's response. For example, in response to a steering fault injection with an intensity of 25%, the safety driver may overcorrect (e.g., 35%) or undercorrect (e.g., 15%).

The AV safety driver training system is also configured to generate, in response to injecting the one or more vehicle operation faults, one or more fault event tags. A fault event tag is an electronically managed data object associated with a particular vehicle operation fault or a particular compound vehicle operation fault. The one or more fault event tags comprise one or more portions of data related to at least one of a fault event tag identifier, the one or more vehicle operation faults, the one or more driver responses executed by the safety driver, the one or more reaction times associated with the driver responses executed by the safety driver, the one or more driver input values, and/or the one or more autonomous driving mode disengagement methods (e.g., a braking input, a steering input, etc.). In various embodiments, once a fault event tag is generated, the fault event tag is stored in an AV safety driver training database. Furthermore, various enterprise personnel (e.g., a safety driver and/or a safety driver trainer) can access the fault event tag (e.g., via a user computing device associated with the AV safety driver training system) for reviewing, updating, commenting, and/or management purposes.

The AV safety driver training system is also configured to generate an AV safety driver training session report. The AV safety driver training session report comprises at least one of one or more fault event tags, one or more portions of image data associated with the safety driver, one or more portions of video data associated with the safety driver, and/or one or more portions of audio data associated with the safety driver. An AV safety driver training session report is associated with a particular safety driver training session related to a particular safety driver and may be comprised of a plurality of digital files (e.g., text files, image files, video files, audio files, and/or the like) related to the results of the particular safety driver training session. In various embodiments, one or more AV safety driver training session reports can be stored in the AV safety driver training database and accessed, updated, deleted, and/or otherwise managed by the AV safety driver training system.

To capture image and/or audio data related to a particular safety driver trainings session, the cockpit of an AV associated with an AV safety driver training system can be outfitted with a driver monitoring kit (DMK). In various embodiments, the DMK comprises one or more image capture devices (e.g., video cameras) and audio recording devices (e.g., microphones) configured to capture image data related to the safety driver as the safety driver performs one or more safety driver training sessions and/or one or more real-world missions associated with a particular enterprise. The DMK can be configured to capture image data related to the hand placement, foot placement, and/or body posture of the safety driver.

Furthermore, the DMK can be configured to capture audio data (e.g., simultaneously with the image data) during the one or more safety driver training sessions and/or the one or more real-world missions. Moreover, the AV safety driver training system can be configured to associate the image and/or audio data captured of a safety driver during a particular safety driver training session and associate the captured image and/or audio data with one or more fault event tags by, for example, matching vehicle operation fault injection timestamp data with timestamp data related to the captured image and/or audio data. As such, the AV safety driver training system can be configured to capture comprehensive data related to the driving patterns (e.g., reaction times, driver responses, body posture, manual driving hand placement, and/or the like) of a particular safety driver for use during one or more subsequent safety driver training sessions.

In various embodiments, AVs associated with the AV safety driver training system can be configured to operate on a specially designed safety track, where the safety track is comprised of real-world roadway features such as signs, obstacles (e.g., other vehicles), markings, and/or the like. For example, the safety track can comprise actual stop signs and/or stop lights such that preconfigured autonomous driving components will cause the AV to operate on the safety track in the same manner as the AV would in the real-world environment upon encountering one or more roadway features. Non-limiting examples of real-world roadway features that can be incorporated on the safety track include, but are not limited to, stop signs, pedestrian crossing signs, railroad crossing signs, street signs, highway markers, traffic signals, lane markings, cross-walk markings, sidewalks, curbs, medians, and/or the like.

Furthermore, the routes associated with the safety track, as well as the corresponding roadway features, can be represented in a digital format and stored in a particular AV (e.g., as map data stored in an onboard map database). As such, various waypoints and/or destinations associated with the safety track can be uploaded into the navigation system associated with the AV such that the AV can be directed to autonomously navigate the safety track during a safety driver training session.

### EXAMPLE SYSTEMS AND APPARATUSES OF THE PRESENT DISCLOSURE

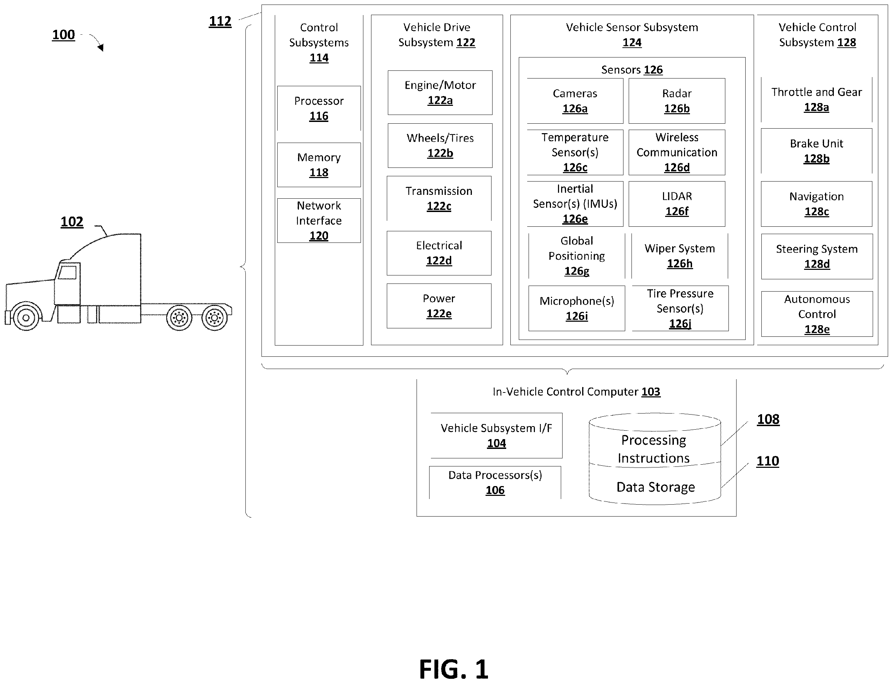

FIG. 1 shows a block diagram of an example vehicle ecosystem 100 in which autonomous driving operations can be determined. As shown in FIG. 1, the AV 102 may be an autonomous semi-truck tractor unit that attaches to a trailer to transport cargo or freight. However, as will be appreciated by one of skilled in the art, the AV 102 may be embodied by any autonomous vehicle such as a car, truck, drone, water vessel, and/or the like. The vehicle ecosystem 100 includes several systems and components that can generate and/or deliver one or more sources of information/data and related services to an in-vehicle control computer 103 that may be located in an AV 102. The in-vehicle control computer 103 can be in data communication with a plurality of AV subsystems 112, all of which can be resident in the AV 102. An AV subsystem interface 104 is provided to facilitate data communication between the in-vehicle control computer 103 and the plurality of AV subsystems 112. In some embodiments, the AV subsystem interface 104 can include a controller area network (CAN) controller to communicate with devices in the AV subsystems 112.

The AV 102 may include various AV subsystems 112 that support the operation of AV 102. The AV subsystems 112 may include the control subsystem 114, a vehicle drive subsystem 122, a vehicle sensor subsystem 124, and/or a vehicle control subsystem 128. The components or devices of the vehicle drive subsystem 122, the vehicle sensor subsystem 124, and the vehicle control subsystem 128 shown in FIG. 1 are examples. The vehicle drive subsystem 122 may include components operable to provide powered motion for the AV 102. In an example embodiment, the vehicle drive subsystem 122 may include an engine/motor 122a, wheels/tires 122b, a transmission 122c, an electrical subsystem 122d, and a power source 122e.

The vehicle sensor subsystem 124 may include a number of sensors 126 configured to sense information about an environment or condition of the AV 102. The vehicle sensor subsystem 124 may include one or more cameras 126a, which may be equipped with microphones, or image capture devices, a radar 126b, one or more temperature sensors 126c, a wireless communication unit 126d (e.g., a cellular communication transceiver), an inertial measurement unit (IMU) 126e, a laser range finder or LiDAR 126f, a Global Positioning System (GPS) transceiver 126g, a wiper control system 126h, microphones 126i, and/or tire pressure sensor(s) 126j. The vehicle sensor subsystem 124 may also include sensors configured to monitor internal systems of the AV 102 (e.g., an 02 monitor, a fuel gauge, an engine oil temperature, etc.).

The IMU 126e may include any combination of sensors (e.g., accelerometers and gyroscopes) configured to sense position and orientation changes of the AV 102 based at least in part on inertial acceleration. The GPS transceiver 126g may be any sensor configured to estimate a geographic location of the AV 102. For this purpose, the GPS transceiver 126g may include a receiver/transmitter operable to provide information regarding the position of the AV 102 with respect to the Earth. The radar 126b may represent a system that utilizes radio signals to sense objects within the local environment of the AV 102. In some embodiments, in addition to sensing the objects, the radar 126b may additionally be configured to sense the speed and the heading of the objects proximate to the AV 102. The laser range finder or LiDAR 126f may be any sensor configured to sense objects in the environment in which the AV 102 is located using lasers. The cameras 126a may include one or more devices configured to capture a plurality of images of the environment of the AV 102. The cameras 126a may be still image cameras or motion video cameras. In some embodiments, the cameras 126a may be configured with one or more microphones to capture audio data. Additionally or alternatively, one or more microphones 126i may be configured to capture audio data of the environment of the AV 102 and/or pertaining to the AV 102.

The vehicle control subsystem 128 may be configured to control the operation of the AV 102 and its components. Accordingly, the vehicle control subsystem 128 may include various elements such as a throttle and gear 128a, a brake unit 128b, a navigation unit 128c, a steering system 128d, and/or an autonomous control unit 128e. The throttle and gear 128a may be configured to control, for instance, the operating speed of the engine and, in turn, control the speed of the AV 102. The throttle and gear 128a may be configured to control the gear selection of the transmission. The brake unit 128b can include any combination of mechanisms configured to decelerate the AV 102. The brake unit 128b can use friction to slow the wheels in a standard manner. The brake unit 128b may include an Anti-lock brake system (ABS) that can prevent the brakes from locking up when the brakes are applied. The navigation unit 128c may be any system configured to determine a driving path or route for the AV 102. The navigation unit 128c may additionally be configured to update the driving path dynamically while the AV 102 is in operation. In some embodiments, the navigation unit 128c may be configured to incorporate data from the GPS transceiver 126g and one or more predetermined maps so as to determine the driving path for the AV 102. The steering system 128d may represent any combination of mechanisms that may be operable to adjust the heading of AV 102 in an autonomous mode or in a driver-controlled mode.

The autonomous control unit 128e may represent a control system configured to identify, evaluate, and avoid or otherwise negotiate potential obstacles or obstructions in the environment of the AV 102. In general, the autonomous control unit 128e may be configured to control the AV 102 for operation without a driver or to provide driver assistance in controlling the AV 102. In some embodiments, the autonomous control unit 128e may be configured to incorporate data from the GPS transceiver 126g, the radar 126b, the LiDAR 126f, the cameras 126a, and/or other AV subsystems 112 to determine the driving path or trajectory for the AV 102.

Many or all of the functions of the AV 102 can be controlled by the in-vehicle control computer 103. The in-vehicle control computer 103 may include at least one data processor 106 (which can include at least one microprocessor) that executes processing instructions 108 stored in a non-transitory computer readable medium, such as the data storage 110 or memory. The in-vehicle control computer 103 may also represent a plurality of computing devices that may serve to control individual components or subsystems of the AV 102 in a distributed fashion. In some embodiments, the data storage 110 may contain processing instructions 108 (e.g., program logic) executable by the data processor 106 to perform various methods and/or functions of the AV 102.

The data storage 110 may contain additional instructions as well, including instructions to transmit data to, receive data from, interact with, or control one or more of the vehicle drive subsystem 122, the vehicle sensor subsystem 124, and the vehicle control subsystem 128. The in-vehicle control computer 103 can be configured to include a data processor 106 and a data storage device 1090. The in-vehicle control computer 103 may control the function of the AV 102 based at least in part on inputs received from various AV subsystems 112 (e.g., the vehicle drive subsystem 122, the vehicle sensor subsystem 124, and the vehicle control subsystem 128).

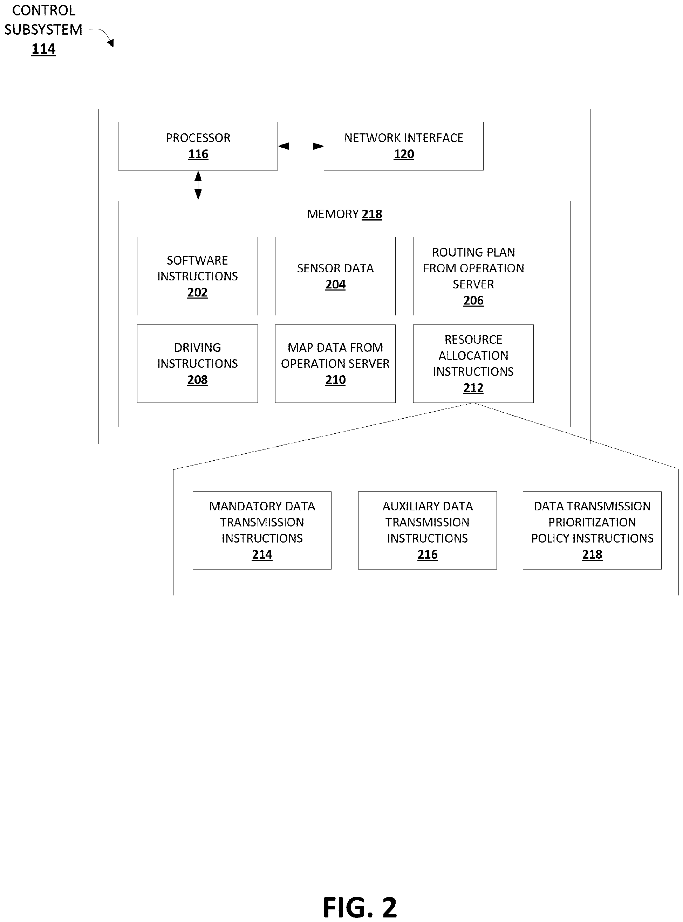

103FIG. 2 illustrates an embodiment of the control subsystem 114. The control subsystem 114 includes at least one processor 116, at least one memory 118, and at least one network interface 120. The control subsystem 114 may be configured as shown or in any other suitable configuration.

The processor 116 may comprise one or more processors operably coupled to the memory 118. The processor 116 is any electronic circuitry including, but not limited to, state machines, one or more central processing unit (CPU) chips, logic units, cores (e.g. a multi-core processor), field-programmable gate arrays (FPGAs), application specific integrated circuits (ASICs), or digital signal processors (DSPs). The processor 116 may be a programmable logic device, a microcontroller, a microprocessor, or any suitable combination of the preceding. The processor 116 is communicatively coupled to and in signal communication with the memory 118 and the network interface 120. The one or more processors are configured to process data and may be implemented in hardware or software. For example, the processor 116 may be 8-bit, 16-bit, 32-bit, 64-bit or of any other suitable architecture. The processor 116 may include an arithmetic logic unit (ALU) for performing arithmetic and logic operations, processor registers that supply operands to the ALU and store the results of ALU operations, and a control unit that fetches instructions from memory and executes them by directing the coordinated operations of the ALU, registers and other components. The one or more processors are configured to implement various instructions. In some embodiments, the function(s) described herein is implemented using logic units, FPGAs, ASICs, DSPs, or any other suitable hardware or electronic circuitry.

The memory 118 is operable to store any of the information described below along with any other data, instructions, logic, rules, or code operable to implement the function(s) described herein when executed by processor 116. For example, the memory 118 may store software instructions 202, sensor data 204 received from the sensors 126, vehicle drive subsystem 122, and/or vehicle control subsystem 128 of the AV 102, map data 210, routing plan 206, driving instructions 208, and/or any other data/instructions described herein. The map data 210 may include a virtual map of a city which includes a route. In some examples, the map data 210 may include one or more map databases. The map data 210 may include drivable areas, such as roads, paths, highways, and undrivable areas, such as terrain. The map data may specify location coordinates of road signs, lanes, lane markings, lane boundaries, road boundaries, traffic lights, etc.

The map data 210 may also specify connections between lanes (e.g., which lanes can feed into other adjacent lanes). The map data 210 may specify information indicating types of lanes of a road (e.g., traffic lane, passing lane, emergency lane, turning lane, bus lane, etc.), types of lane boundaries (e.g., white lines, yellow lines, other road surface markings and/or mechanical markings, etc.), types of road boundaries (e.g., regular curbs, red curbs, sidewalks, guard rails, other barriers, etc.), road intersections, one or more obstacles ahead of the autonomous vehicle, and other information about the road or areas adjacent to the road. The map data 210 may also specify elevations of roads, such as curves, hills, valleys; road hazards, such as speed bumps, potholes; road sections, such as road school zones, railroad crossings, etc.

In an example embodiment, the map data 210 may also comprise data related to a safety track configured for training safety drivers associated with an AV fleet. For example, in some embodiments, a safety track may be comprised of real-world roadway features such as signs, obstacles (e.g., other vehicles), markings, and/or the like. The safety track can comprise actual stop signs and/or stop lights such that preconfigured autonomous driving components will cause the AV to operate on the safety track in the same manner as the AV would in the real-world environment upon encountering one or more roadway features. Non-limiting examples of real-world roadway features that can be incorporated on the safety track and represented in the map data 210 may include, but are not limited to, stop signs, pedestrian crossing signs, railroad crossing signs, street signs, highway markers, traffic signals, lane markings, cross-walk markings, sidewalks, curbs, medians, and/or the like.

The routing plan 206 is a plan for traveling from a start location (e.g., a first AV launch pad/landing pad) to a destination (e.g., a second AV launchpad/landing pad). For example, the routing plan 206 of the AV 102 may specify a combination of one or more streets/roads/highways in a specific order from the start location to the destination. The routing plan 206 of the AV 102 may specify stages including the first stage (e.g., moving out from the start location), a plurality of intermediate stages (e.g., traveling along particular lanes of one or more particular street/road/highway), and the last stage (e.g., entering the destination). The routing plan 206 may include other information about the route from the start position to the destination, such as road/traffic signs in that routing plan 206, etc.

The driving instructions 208 may include instructions and rules to adapt the autonomous driving of the AV 102 according to the driving rules of each stage of the routing plan. For example, the driving instructions 208 may include instructions to stay within the speed range of a road traveled by the AV 102, adapt the speed of the AV 102 with respect to observed changes by the sensors 126, such as speeds of surrounding vehicles, objects within the detection zones of the sensors, etc.

The memory 118 may further store resource allocation instructions 212, which may be configured to determine mandatory data transmission instructions 214, auxiliary data transmission instructions 216 and data transmission prioritization policy instructions 218. The memory 118 may comprise one or more disks, tape drives, or solid-state drives, and may be used as an over-flow data storage device, to store programs when such programs are selected for execution, and to store instructions and data that are read during program execution. The memory 118 may be volatile or non-volatile and may comprise read-only memory (ROM), random-access memory (RAM), ternary content-addressable memory (TCAM), dynamic random-access memory (DRAM), and static random-access memory (SRAM).

The network interface 120 is configured to enable wired and/or wireless communications. The network interface 120 is configured to communicate data between the control subsystem 114 and other network devices, systems, or domain(s). For example, the network interface 120 may comprise a WIFI interface, a local area network (LAN) interface, a wide area network (WAN) interface, a modem, a switch, or a router. The processor 116 is configured to send and receive data using the network interface 120. The network interface 120 may be configured to use any suitable type of communication protocol.

In one embodiment, the control subsystem 114 may be a subsystem of the in-vehicle control computer 103 (See FIG. 1). The control subsystem 114 may be implemented by one or more computing devices that may serve to determine a data transmission prioritization policy and/or control the transmission of mandatory data and/or auxiliary data. The control subsystem 114 is in signal communication with the in-vehicle control computer 103 (and its components).

In some embodiments, the mandatory data transmission instructions 214 may be configured to provide instructions regarding the transmission of mandatory data from the AV 102. The instructions may be indicative of which mandatory data to transmit, a data pre-processing policy indicative of how mandatory data should be processed prior to being transmitted from the AV 102, a frequency for which the mandatory data is to be transmitted from the AV 102, receiving computing entities, and/or the like. The mandatory data transmission instructions 214 may be configured to cause a mandatory data object of mandatory data to be transmitted from the AV 102 to one or more computing entities.

In some embodiments, the auxiliary data transmission instructions 216 may be configured to provide instructions regarding the transmission of auxiliary data from the AV 102. The instructions may be indicative of which auxiliary data to transmit, a data pre-processing policy indicative of how auxiliary data should be processed prior to being transmitted from the AV 102, a frequency for which the auxiliary data is to be transmitted from the AV 102, receiving computing entities, a measure of auxiliary communication network resource availability, and/or the like. The auxiliary data transmission instructions 216 may be configured to cause an auxiliary data object of auxiliary data to be transmitted from the AV 102 to one or more computing entities.

In some embodiments, data transmission prioritization policy instructions 218 may be configured to provide instructions regarding the determination of a data transmission prioritization policy. For example, the data transmission prioritization policy instructions 218 may provide instructions regarding the determination of one or more operational states associated with the AV 102, generation of the data transmission prioritization policy, updating of the transmission prioritization policy, and/or the like. The data transmission prioritization policy may be used by the auxiliary data transmission instructions 216 with regard to the transmission of auxiliary data.

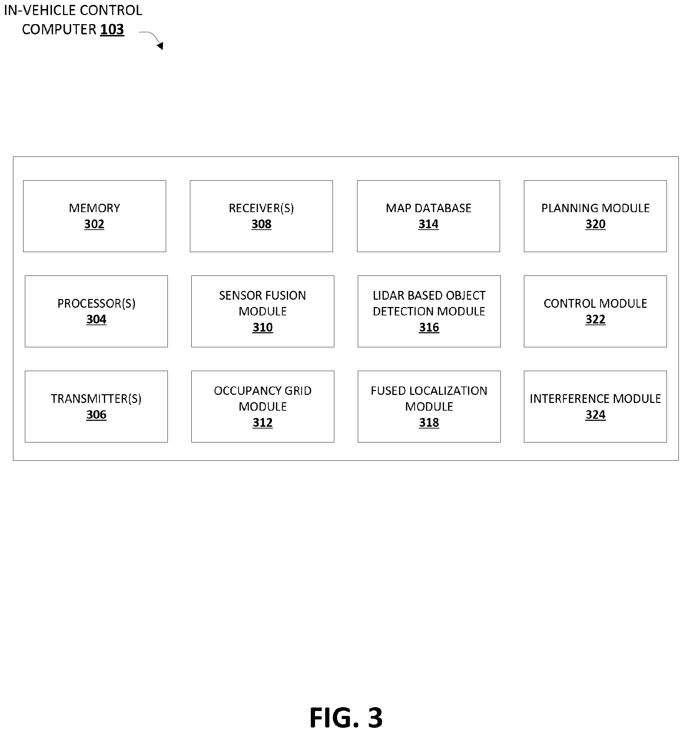

FIG. 3 shows an exemplary block diagram of an in-vehicle control computer 103 included in an AV 102. The in-vehicle control computer 103 includes at least one processor 304 and a memory 302 having instructions stored thereupon (e.g., processing instructions 108 and software instructions 202, and in FIGS. 1 and 2, respectively). The instructions upon execution by the processor 304 configure the in-vehicle control computer 103 and/or the various modules of the in-vehicle control computer 103 to perform the operations described in FIGS. 1 and 2. The transmitter 306 transmits or sends information or data to one or more devices in the autonomous vehicle. For example, a transmitter 306 can send an instruction to one or more motors of the steering wheel to steer the autonomous vehicle. The receiver 308 receives information or data transmitted or sent by one or more devices. For example, the receiver 308 receives a status of the current speed from the odometer sensor or the current transmission gear from the transmission. The transmitter 306 and receiver 308 are also configured to communicate with the control subsystem 114.

The in-vehicle control computer 103 may include a sensor fusion module 310, which may perform image or signal processing operations. The sensor fusion module 310 can obtain images from cameras located on an autonomous vehicle to perform image segmentation to detect the presence of moving objects (e.g., other vehicles, pedestrians, etc.,) and/or static obstacles (e.g., a stop sign, speed bump, terrain, etc.,) located around the autonomous vehicle. The sensor fusion module 310 can obtain LiDAR point cloud data item from LiDAR sensors located on the autonomous vehicle to perform LiDAR segmentation to detect the presence of objects and/or obstacles located around the autonomous vehicle.

The sensor fusion module 310 can perform instance segmentation on an image and/or a point cloud data item to identify an outline (e.g., boxes) around the objects and/or obstacles located around the autonomous vehicle. The sensor fusion module 310 can perform temporal fusion where objects and/or obstacles from one image and/or one frame of point cloud data item are correlated with or associated with objects and/or obstacles from one or more images or frames subsequently received in time.

The sensor fusion module 310 can fuse the objects and/or obstacles from the images obtained from the camera and/or point cloud data item obtained from the LiDAR sensors. For example, the sensor fusion module 310 may determine based on a location of two cameras that an image from one of the cameras comprising one half of a vehicle located in front of the autonomous vehicle is the same as the vehicle captured by another camera. The sensor fusion module 310 sends the fused object information to the interference module 324 and the fused obstacle information to the occupancy grid module 312. The in-vehicle control computer includes the occupancy grid module 312 that can retrieve landmarks from a map database 314 stored in the in-vehicle control computer. The occupancy grid module 312 can determine drivable areas and/or obstacles from the fused obstacles obtained from the sensor fusion module 310 and the landmarks stored in the map database 314. For example, the occupancy grid module 312 can determine that a drivable area may include a speed bump obstacle.

The in-vehicle control computer 103 may also include a LiDAR based object detection module 316 that can perform object detection based on a point cloud data item obtained from the LiDAR sensors located on the AV 102. The object detection technique can provide a location (e.g., in 3D world coordinates) of objects from the point cloud data item. Below the LiDAR based object detection module 316, the in-vehicle control computer may include an image based object detection module that can perform object detection based on images obtained from cameras located on the autonomous vehicle. The object detection technique can employ a deep machine learning technique to provide a location (e.g., in 3D world coordinates) of objects from the image provided by the camera.

The radar on the autonomous vehicle can scan an area in front of the autonomous vehicle or an area towards which the autonomous vehicle is driven. The radar data is sent to the sensor fusion module 310 that can use the radar data to correlate the objects and/or obstacles detected by the radar with the objects and/or obstacles detected from both the LiDAR point cloud data item and the camera image.

The planning module 320 can perform navigation planning to determine a set of trajectories on which the AV 102 can be driven. The set of trajectories can be determined based on the drivable area information, location of the obstacles, and the drivable area information. In some embodiments, the navigation planning may include determining an area next to the road where the autonomous vehicle can be safely parked in case of emergencies. The planning module 320 may include behavioral decision making to determine driving actions (e.g., steering, braking, throttle) in response to determining changing conditions on the road (e.g., traffic light turned yellow, or the autonomous vehicle is in an unsafe driving condition because another vehicle drove in front of the autonomous vehicle and in a region within a pre-determined safe distance of the location of the autonomous vehicle). The planning module 320 performs trajectory generation and selects a trajectory from the set of trajectories determined by the navigation planning operation. The selected trajectory information is sent by the planning module 320 to the control module 322.

The in-vehicle control computer includes a control module 322 that receives the proposed trajectory from the planning module 320 and the autonomous vehicle location and pose from the fused localization module 318. The control module 322 includes a system identifier. The control module 322 can perform a model based trajectory refinement to refine the proposed trajectory. For example, the control module 322 can apply a filter (e.g., Kalman filter) to make the proposed trajectory data smooth and/or to minimize noise. The control module 322 may perform the robust control by determining, based on the refined proposed trajectory information and current location and/or pose of the autonomous vehicle, an amount of brake pressure to apply, a steering angle, a throttle amount to control the speed of the vehicle, and/or a transmission gear. The control module 322 can send the determined brake pressure, steering angle, throttle amount, and/or transmission gear to one or more devices in the autonomous vehicle to control and facilitate precise driving operations of the autonomous vehicle.

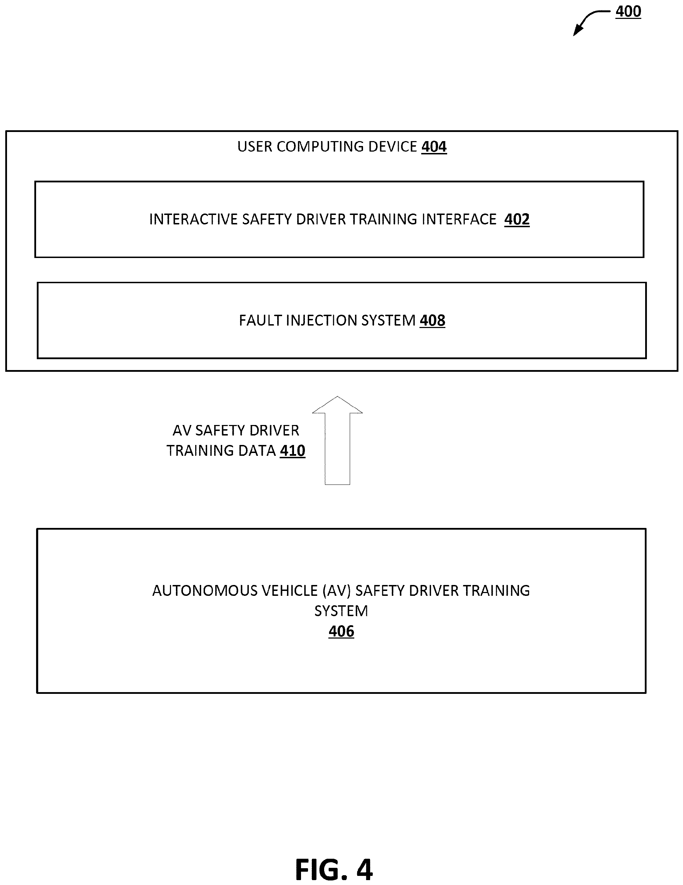

FIG. 4 illustrates an exemplary system 400 associated with an interactive safety driver training interface 402, in accordance with some embodiments discussed in the present disclosure. In an embodiment, the system 400 includes a user computing device 404 and a AV safety driver training system 406. In one or more embodiments, the user computing device 404 includes a user computing device system 408 to facilitate generation of an interactive safety driver training interface 402. In one or more embodiments, the interactive safety driver training interface 402 is rendered via a visual display 604 of the user computing device system 408. Furthermore, in certain embodiments, the AV safety driver training system 406 is communicatively coupled not only to the user computing device 404, but to the in-vehicle control computer 103 associated with an AV 102.

The interactive safety driver training interface 402 is associated with a dashboard visualization service (e.g., an AV safety driver training service) rendered via a display of a user computing device 404. In one or more embodiments, the interactive safety driver training interface 402 is configured to provide one or more vehicle operation fault injection selection configurations associated with AV safety driver training data 410. In one or more embodiments, the AV safety driver training system 406 is configured to provide the AV safety driver training data 410to the user computing device 404 to facilitate rendering of the interactive safety driver training interface 402 related to the AV 102 and/or one or more AV safety driver training sessions.

In one or more embodiments, the interactive safety driver training interface 402 is configured to provide dynamic interaction with vehicle operation fault injection configuration options that are rendered as respective interactive display elements via the interactive safety driver training interface 402. An interactive display element is a portion of the interactive safety driver training interface (e.g., a user-interactive electronic interface portion) that provides interaction with respect to an end user of the user computing device 404. For example, in one or more embodiments, an interactive display element is an interactive display element that allows a user to provide feedback and/or to perform one or more actions with respect to the interactive safety driver training interface 402. Non-limiting examples of interactive display elements can include interactive buttons, sliders, hyperlinks, text input fields, graphs, charts, tables, and/or the like.

In an embodiment, in response to user interaction with an interactive display element, the interactive safety driver training interface 402 is dynamically altered to display one or more altered portions of the interactive safety driver training interface 402 associated with different visual data and/or different interactive display elements. Additionally, in one or more embodiments, the interactive safety driver training interface 402 is configured to facilitate execution and/or initiation of one or more actions via the dashboard visualization, such as via an interactive display element of the dashboard visualization, based on the AV safety driver training data. In certain embodiments, the interactive safety driver training interface 402 is configured to present one or more notifications associated with the prioritized actions related to the AV safety driver training data 410.

The interactive safety driver training interface 402 of an example embodiment is configured to display respective interactive display elements associated with the AV safety driver training data 410. In one or more embodiments, the visual display 604 of the user computing device system 408 is configured to provide the interactive safety driver training interface that is configured to allow a user associated with the user computing device 404 to interact with respective user interface configurations of the interactive safety driver training interface. Additionally, in one or more embodiments, the visual display 604 of the user computing device 404 is configured to provide the interactive safety driver training interface 402 that is configured to allow a user associated with the user computing device 404 to control the one or more interactive display elements associated with AV safety driver training data 410. In one or more embodiments, the interactive safety driver training interface is configured based on user profile data and/or user privileges. In certain embodiments, user specific requirements associated with the interactive safety driver training interface 402 is configured via a backend system (e.g., the AV safety driver training system 406). In one or more embodiments, the interactive safety driver training interface 402 is configured based on hardware and/or software specifications of the user computing device 404.

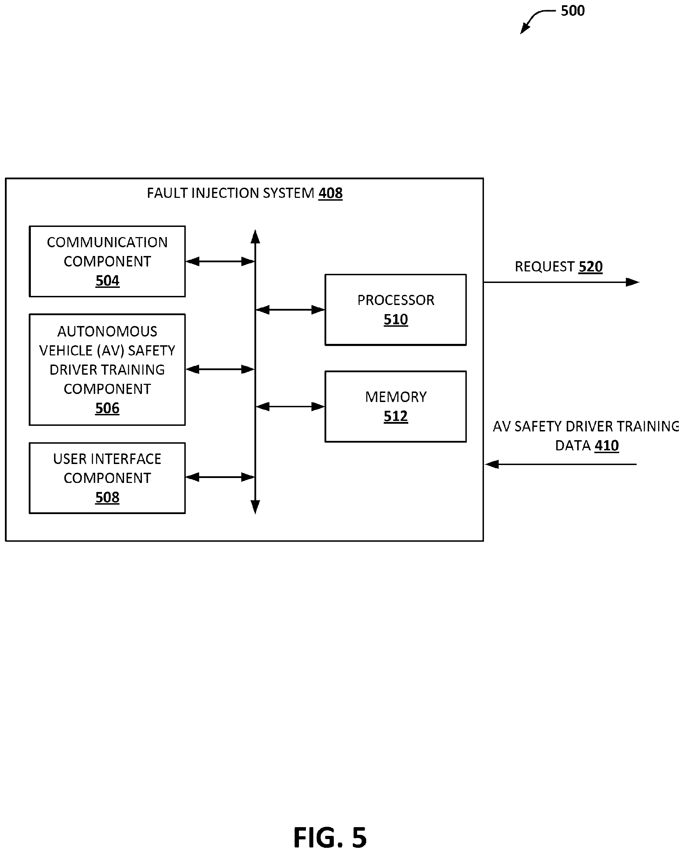

FIG. 5 illustrates a system 500 that includes the fault injection system 408 discussed above in relation to the system of FIG. 4 and configured to provide a practical application of facilitating vehicle operation fault injection for an AV 102, such as an AV 102 associated with an AV fleet. In one or more embodiments, the fault injection system 408 provides a practical application of rendering AV safety driver training data 410 associated with the AV 102. In one or more embodiments, the fault injection device system 408 employs the AV safety driver training data 410 that is aggregated from an AV safety driver training session associated with a particular AV 102 to provide insights, such as in real-time or near real-time, related to the AV safety driver training session.

In an embodiment, the fault injection system 408 facilitates interaction with an autonomous vehicle network platform, such as may be associated with one or more data sources, and/or an AV 102. In one or more embodiments, the fault injection system 408 is configured to provide the interactive safety driver training interface 402 associated with vehicle operation fault injections related to an AV safety driver training session associated with an AV 102 and a particular safety driver.

As noted above, the interactive safety driver training interface 402 may be configured as a dashboard visualization associated with an AV 102, where the visualization data comprises one or more pieces of data related to, but not limited by, AV safety driver training data, data related to an AV safety driver training session report, vehicle operation fault injection data, compound vehicle operation fault injection data, AV identification data, AV subsystem data (e.g., data related to control subsystem 114, vehicle drive subsystem 122, vehicle sensor subsystem 124, vehicle control subsystem 128), AV location data, mission data, AV log data, routing data (e.g., routing plan 206), AV trailer data, AV cargo data, and/or the like. In one or more embodiments, the fault injection system 408 interacts with an in-vehicle control computer 103 associated with a particular AV 102 in order to cause execution of one or more commands by the in-vehicle control computer 103. Additionally, the fault injection system 408 can access data related to the one or more AV subsystems 112 associated with an AV 102 via the in-vehicle control computer 103, the data processor(s) 106, and/or the data storage 110.

The fault injection system 408 may also include a communication component 504, an AV safety driver training component 506 and/or a user interface component 508. Additionally, in one or more embodiments, the fault injection system 408 includes a processor 510 and/or a memory 512. In certain embodiments, one or more aspects of the fault injection system 408 (and/or other systems, apparatuses and/or processes disclosed herein) constitute executable instructions embodied within a computer-readable storage medium (e.g., the memory 512). For instance, in an embodiment, the memory 512 stores computer executable components and/or executable instructions (e.g., program instructions). Furthermore, the processor 510 facilitates execution of the computer executable components and/or the executable instructions (e.g., the program instructions). In an example embodiment, the processor 510 is configured to execute instructions stored in the memory 512 or otherwise accessible to the processor 510.

The processor 510 of an example embodiment is a hardware entity (e.g., physically embodied in circuitry) capable of performing operations according to one or more embodiments of the disclosure. Alternatively, in an embodiment where the processor 510 is embodied as an executor of software instructions, the software instructions configure the processor 510 to perform one or more algorithms and/or operations described herein in response to the software instructions being executed. In an embodiment, the processor 510 is a single core processor, a multi-core processor, multiple processors internal to the user computing device system 408, a remote processor (e.g., a processor implemented on a server), and/or a virtual machine. In certain embodiments, the processor 510 is in communication with the memory 512, the communication component 504, the AV safety driver training component 506 and/or the user interface component 508 via a bus to, for example, facilitate transmission of data among the processor 510, the memory 512, the communication component 504, the AV safety driver training component 506 and/or the user interface component 508. The processor 510 may be embodied in a number of different ways and, in certain embodiments, includes one or more processing devices configured to perform independently. Additionally or alternatively, in one or more embodiments, the processor 510 includes one or more processors configured in tandem via a bus to enable independent execution of instructions, pipelining of data, and/or multi-thread execution of instructions.

The memory 512 is non-transitory and includes, for example, one or more volatile memories and/or one or more non-volatile memories. In other words, in one or more embodiments, the memory 512 is an electronic storage device (e.g., a computer-readable storage medium). The memory 512 is configured to store information, data, content, one or more applications, one or more instructions, or the like, to enable the fault injection system 408 to carry out various functions in accordance with one or more embodiments disclosed herein.

In one or more embodiments, the communication component 504 is configured to generate a request 520. In various contexts, the request 520 may be a request to obtain AV safety driver training data 410 associated with an AV 102 operating in an AV fleet managed by a particular enterprise. In various other contexts, the request 520 may be a request to generate and/or inject one or more vehicle operation faults and/or one or more compound vehicle operation faults for a particular AV 102. In various embodiments, the communication component 504 generates the request 520 in response to an action performed with respect to a first user interface configuration for the interactive safety driver training interface 402. The action can be, for example, initiating execution of an application (e.g., a mobile application) via the user computing device 404 that presents the interactive safety driver training interface 402, altering an interactive graphical element via the interactive safety driver training interface 402, or taking another type of action with respect to the interactive safety driver training interface 402. Additionally or alternatively, in one or more embodiments, the communication component 504 generates the request 520 in response to execution of a user authentication process via the user computing device 404. For example, in an embodiment, the user authentication process is associated with password entry, facial recognition, biometric recognition, security key exchange, and/or another security technique associated with the user computing device 404.

In various embodiments, the interactive safety driver training interface 402 is a dashboard visualization related to various AV safety driver training protocols to be executed in relation to the safety driver associated with the AV 102. In one or more embodiments, the request 520 includes one or more AV identifiers related to the AV 102. In some embodiments, various AV identifiers (IDs) can include, but are not limited to, truck IDs, trailer IDs, load IDs, car IDs, SUV IDs, cargo Ids, and/or the like. In various embodiments, AV identifiers can be generated by the AV safety driver training system 406. In one or more embodiments, one or more AV identifiers can be associated with one or more vehicle identifiers issued by a governing body, such as, for example, the Department of Motor Vehicles (DMV). Such vehicle identifiers can include, but are not limited to, license plate numbers, trailer numbers, vehicle identification numbers (VINs), and/or any other various identifiers associated with an AV 102 that has been issued by a regulatory body.

Additionally or alternatively, in one or more embodiments, the request 520 includes one or more user identifiers describing a user role for a user profile associated with the user computing device 404 accessing the AV safety driver training system 406. A user identifier includes, for example, an identifier for a user profile, where the user profile can be associated with a particular user role. Various user roles can include, but are not limited to, a safety driver, a safety driver trainer, an administrator, an operator, and/or a third-party that might have a reason to interact with an AV 102 and/or the AV safety driver training system 406, such as regulatory personnel.

In various embodiments, a user role that is associated with a particular user profile can affect the availability of various permissions, configurations, and/or actions associated with the AV safety driver training system 406 for the user profile associated with the respective user role. Additionally, the AV safety driver training system 406 can be configured to determine certain configurations and parameters associated with one or more AV safety driver training sessions and/or one or more vehicle operation faults related to a safety driver associated with an AV 102. Additionally, the AV safety driver training system 406 can generate one or more interactive AV safety driver training session reports based on (and differing depending upon) the user role associated with a respective user profile (e.g., a safety driver profile or a safety driver trainer profile).

In an embodiment, the communication component 504 is configured to transmit the request 520. In one or more embodiments, the communication component 504 transmits the request 520, such as via a network, to a server system, such as the AV safety driver training system 406. In response to the request 520, the communication component 504 and/or the AV safety driver training component 506 is configured to receive AV safety driver training data 410, such as via the network, to facilitate altering configuration of the interactive safety driver training interface 402 based on the AV safety driver training data 410. In one or more embodiments, the AV safety driver training component 506 is configured to receive the AV safety driver training data 410 from the server system, such as the AV safety driver training system 406.

In certain embodiments, the communication component 504 and/or the AV safety driver training component 506 incorporates encryption capabilities to facilitate encryption and/or decryption of one or more portions of the AV safety driver training data 410. In one or more embodiments, the network 702 via which the request 520 is transmitted and the AV safety driver training data 410 is received, may be any of a variety of networks, such as a Wi-Fi network, a cellular network, a Near Field Communications (NFC) network, a Worldwide Interoperability for Microwave Access (WiMAX) network, a personal area network (PAN), a short-range wireless network (e.g., a Bluetooth^{®} network), an infrared wireless (e.g., IrDA) network, an ultra-wideband (UWB) network, an induction wireless transmission network, and/or another type of network.

In one or more embodiments, the AV safety driver training data 410 is configured based on the one or more AV identifiers and/or the one or more user identifiers. Additionally, in one or more embodiments, the AV safety driver training data 410 is configured based on one or more enterprise-defined AV safety driver training protocols and/or a particular AV safety driver training session. For example, the AV safety driver training data 410 may comprise data related to, but not limited by, one or more vehicle operation faults, one or more compound vehicle operation faults, one or more driver responses, one or more driver reaction times, one or more driver input values, one or more autonomous driving mode disengagement methods, one or more fault event tags, and/or one or more AV safety driver training session reports.

The user interface component 508 is configured to render the interactive safety driver training interface 402 via a display of the user computing device 404. In one or more embodiments, the interactive safety driver training interface 402 is configured as a dashboard visualization rendered via a display of a user computing device. As described above, the interactive safety driver training interface 402 of an example embodiment is configured to provide dynamic interaction with vehicle operation fault configurations that are rendered as respective interactive display elements via the interactive safety driver training interface 402. An interactive display element is a portion of the interactive safety driver training interface (e.g., a user-interactive electronic interface portion) that provides interaction with respect to an end user of the user computing device 404. For example, in one or more embodiments, an interactive display element is an interactive display element that allows a user to provide feedback and/or to perform one or more actions with respect to the interactive safety driver training interface 402. Non-limiting examples of interactive display elements can include interactive buttons, sliders, hyperlinks, text input fields, graphs, charts, and/or tables.

In an embodiment, in response to interaction with an interactive display element, the interactive safety driver training interface 402 is caused to be dynamically altered to display one or more altered portions of the interactive safety driver training interface 402 associated with different visual data and/or different interactive display elements. Additionally, in one or more embodiments, the interactive safety driver training interface 402 is configured to facilitate the execution and/or initiation of one or more actions via the dashboard visualization based on the AV safety driver training data 410 (e.g., initiation of a vehicle operation fault). In an embodiment, an action is executed and/or initiated via an interactive display element of the dashboard visualization. In certain embodiments, the interactive safety driver training interface 402 presents one or more notifications associated with the prioritized actions related to the AV safety driver training data 410.

In one or more embodiments, the AV safety driver training component 506 interfaces with the user interface component 508 to alter a first user interface configuration for the interactive safety driver training interface 402 based on the AV safety driver training data 410. For example, in one or more embodiments, the AV safety driver training component 506 is configured to alter the first user interface configuration for the interactive safety driver training interface 402 based on the AV safety driver training data 410 to provide a second user interface configuration for the interactive safety driver training interface 402. Additionally, the user interface component 508 can render the second user interface configuration for the interactive safety driver training interface 402 via the display of the user computing device 404. In one or more embodiments, the second user interface configuration includes respective interactive display elements related to the one or more vehicle operation fault configurations and/or one or more user input fields. Additionally, in one or more embodiments, the user interface component 508 renders the respective interactive display elements for the second user interface configuration via the interactive safety driver training interface 402 based on the AV safety driver training data 410.

Additionally, the user interface component 508 may be configured to arrange the respective interactive display elements related to the one or more vehicle operation faults based on an enterprise-defined AV safety driver training protocol. The enterprise-defined AV safety driver training protocol can be a predetermined sequence and/or ordering of a plurality of vehicle operation faults and/or compound vehicle operation faults associated with a respective AV safety driver training session. Additionally, the enterprise-defined AV safety driver training protocol can be updated, re-sequenced, and/or re-ordered at any time by way of the fault injection system 408. For example, the user interface component 508 can configure a plurality of vehicle operation fault data objects as interactive display elements on the interactive safety driver training interface 402 such that an end user (e.g., a safety driver trainer) can manipulate the vehicle operation fault data objects, thereby rearranging, re-sequencing, re-ordering, and/or updating the corresponding enterprise-defined AV safety driver training protocol.

In certain embodiments, the user interface component 508 generates one or more interactive display elements comprising a real-time status of updates to the AV 102. The one or more interactive display elements comprising a real-time status of the AV 102 can be based on one or more respective portions of AV safety driver training data 410. For example, the real-time status of updates can be related to a vehicle operation fault injection, a driver response, a driver reaction time, a vehicle operation fault injection duration, an autonomous driving mode disengagement method, and/or the like. Additionally, in response to an interaction with respect to the interactive display elements, the user interface component 508 can alter the interface configuration for the interactive safety driver training interface 402 to display one or more portions of data related to a particular AV safety driver training session, such as, for example, image data, audio data, and/or the like related to the AV safety driver training session.

In certain embodiments, the fault injection system 408, by way of the user interface component 508, is configured to provide remote control of an AV 102. For example, based on an interaction with the interactive safety driver training interface 402, the fault injection system 408 can transmit one or more commands to an in-vehicle control computer 103 associated with a particular AV 102. A non-limiting example of a command that can be issued by the fault injection system 408 includes commands that cause the AV 102 to inject a vehicle operation fault associated with one or more components of a respective AV subsystem. Additionally, the fault injection system 408 can issue a command to deploy an AV 102 to a particular destination (e.g., a destination associated with the safety track) upon successful completion of one or more vehicle operation fault injections and/or driver responses to one or more vehicle operation fault injections associated with the AV 102.

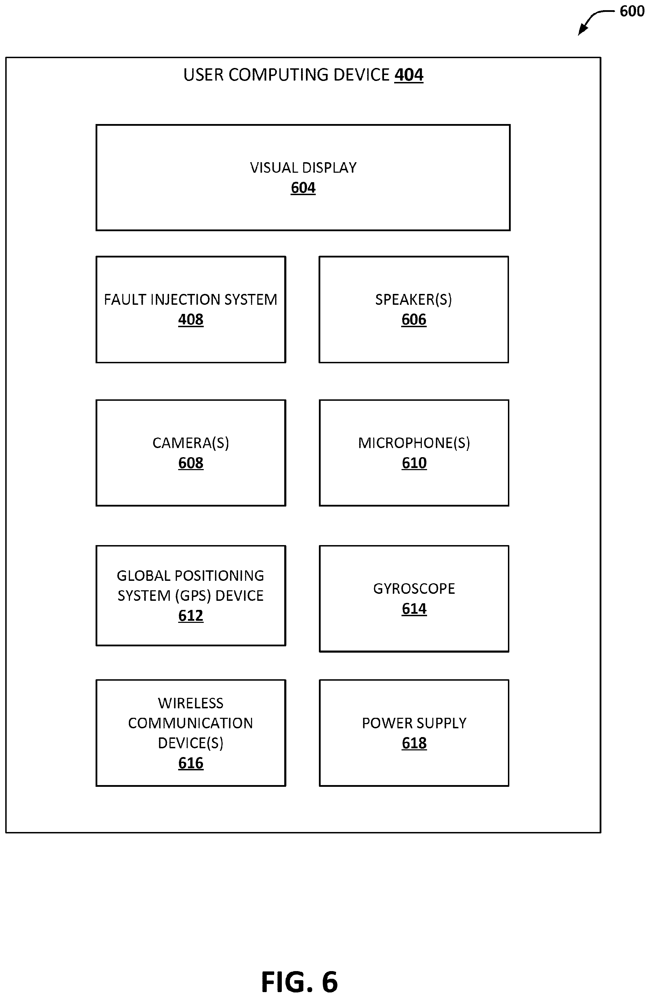

FIG. 6 illustrates an exemplary user computing device in accordance with some embodiments discussed herein. Specifically, FIG. 6 illustrates a system 600 including the user computing device 404. The user computing device 404 can be a mobile computing device, a smartphone, a tablet computer, a mobile computer, a desktop computer, a laptop computer, a workstation computer, a wearable device, a virtual reality device, an augmented reality device, or another type of computing device. The user computing device 404 includes the fault injection system 408 as described above in conjunction with FIG. 5. In the embodiment shown in FIG. 6, the user computing device 404 also includes a visual display 604, one or more speakers 606, one or more cameras 608, one or more microphones 610, a global positioning system (GPS) device 612, a gyroscope 614, one or more wireless communication devices 616, and/or a power supply 618. However, in other embodiments, the user computing device 404 need not include all of these additional components.

In an embodiment, the visual display 604 is a display that facilitates presentation of and/or interaction with the dashboard visualization associated with the one or more vehicle operation faults, driver responses, and/or driver input values associated with AV safety driver training data 410. In one or more embodiments, the user computing device 404 displays the interactive safety driver training interface. In one or more embodiments, the visual display 604 is a visual display that renders data associated with the interactive safety driver training interface 402. In one or more embodiments, the visual display 604 displays respective interactive display elements associated with the AV safety driver training data 410. In one or more embodiments, the visual display 604 provides the interactive safety driver training interface 402 that is configured to allow a user associated with the user computing device 404 to interact with respective user interface configurations of the interactive safety driver training interface 402. Additionally, in one or more embodiments, the visual display 604 provides the interactive safety driver training interface 402 that is configured to allow a user associated with the user computing device 404 to control the one or more interactive display elements associated with one or more vehicle operation faults and/or one or more compound vehicle operation faults. In one or more embodiments, the interactive safety driver training interface 402 is configured based on user profile data and/or user privileges. In certain embodiments, user specific requirements associated with the interactive safety driver training interface 402 is configured via a backend system (e.g., an AV safety driver training system). In one or more embodiments, the interactive safety driver training interface 402 is configured based on hardware and/or software specifications of the user computing device 404.

The one or more speakers 606 include one or more integrated speakers that project audio. The one or more cameras 608 include one or more cameras that employ autofocus and/or image stabilization for photo capture and/or real-time video. The one or more microphones 610 include one or more digital microphones that employ active noise cancellation to capture audio data. The GPS device 612 provides a geographic location for the user computing device 404. The gyroscope 614 provides an orientation for the user computing device 404. The one or more wireless communication devices 616 includes one or more hardware components to provide wireless communication via one or more wireless networking technologies and/or one or more short-wavelength wireless technologies. The power supply 618 may be, for example, a rechargeable battery or other type of power source that provides power to the visual display 604, the one or more speakers 606, the one or more cameras 608, the one or more microphones 610, the GPS device 612, the gyroscope 614, and/or the one or more wireless communication devices 616. In certain embodiments, the AV safety driver training data 410 is presented via the visual display 604 and/or the one or more speakers 606. In certain embodiments, the visual display 604, the one or more cameras 608, the one or more microphones 610, and/or the GPS device 612 facilitate the user authentication process. In certain embodiments, the one or more wireless communication devices 616 are configured via the communication component 504 to facilitate transmission of the request 520 and receipt of the AV safety driver training data 410.

In various embodiments, the camera(s) 608 and/or the microphone(s) 610 are associated with a driver monitoring kit (DMK). In various embodiments, the DMK comprises a plurality of image capture devices (e.g., video cameras) and/or audio recording devices (e.g., microphones) configured to capture image and audio data related to the safety driver as the safety driver performs one or more safety driver training sessions and/or one or more real-world missions associated with a particular enterprise. The DMK can be configured to capture image data related to the hand placement, foot placement, head orientation and/or body posture of the safety driver as well as movement of the hands, feet, head and body of the safety driver.

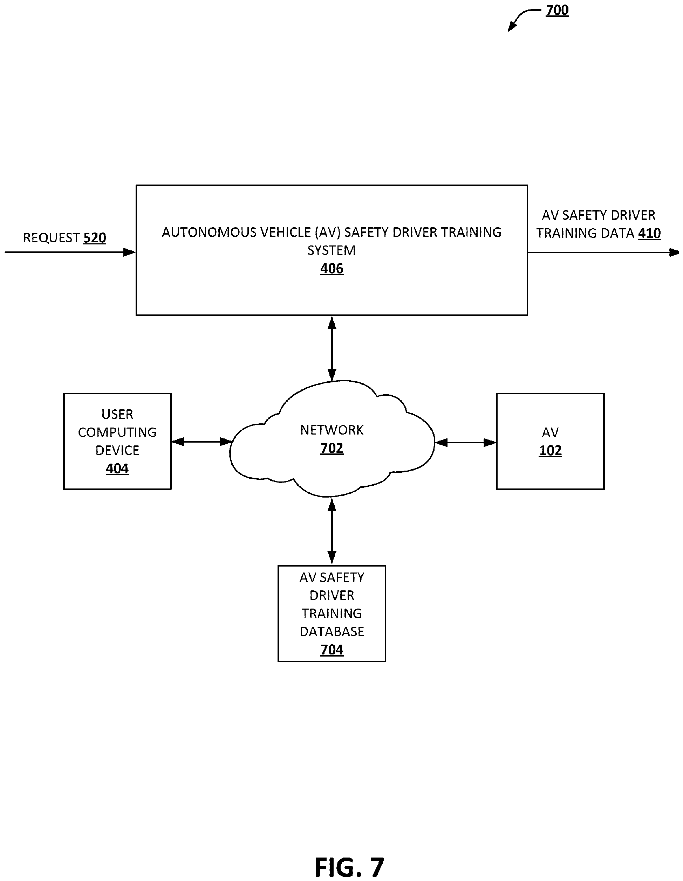

FIG. 7 illustrates a system 700 that includes the AV safety driver training system 406. In one or more embodiments, the AV safety driver training system 406 receives the request 520 from the fault injection system 408 of the user computing device 404 following transmission via, for example, the network 702. Additionally, in one or more embodiments, the AV safety driver training system 406 transmits the AV safety driver training data 410, such as via the network 702, to the fault injection system 408 of the user computing device 404. Additionally, in one or more embodiments, the user computing device 404 also includes the visual display 604, the one or more speakers 606, the one or more cameras 608, the one or more microphones 610, the GPS device 612, the gyroscope 614, the one or more wireless communication devices 616, and/or the power supply 618.

In various embodiments, the AV safety driver training system 406 receives data from multiple sources including, but not limited to, the in-vehicle control computer 103 (e.g., from the data storage 110 comprised in the in-vehicle control computer 103), the user computing device 404, and/or the AV 102. In one or more embodiments, at least a portion of the data from the in-vehicle control computer 103, the user computing device 404, and/or the AV 102 is included in the AV safety driver training data 410. In one or more embodiments, the 103AV 102 is associated with a fleet of AVs 102 owned and operated by a particular enterprise. For example, the fleet of AVs 102 can be associated with an enterprise including, but not limited to, a transportation enterprise, a logistics enterprise, a freight delivery enterprise, a retail enterprise, a manufacturing enterprise, an agricultural enterprise, an energy production enterprise, a travel enterprise, and/or any other type of relevant enterprise.

In one or more embodiments, the AV safety driver training system 406 receives data pertaining to one or more AV subsystems including, but not limited to, the control subsystem 114, vehicle drive subsystem 122, vehicle sensor subsystem 124, vehicle control subsystem 128 and each of the respective components comprised therein. The AV safety driver training system 406 can also receive data pertaining to one or more physical parts of an AV 102 not necessarily comprised in one of the named AV subsystems from above such as, for example, any physical components associated with the AV 102 such as mirrors, windows, doors, etc. Additionally, the AV safety driver training system 406 can receive data associated with any trailers and/or cargo associated with a particular AV 102. Additionally, the AV safety driver training system 406 may be configured to receive data associated with any personnel related to a particular enterprise responsible for a particular AV 102 such as, for example, safety drivers, safety driver trainers, operators, ground crew, managers, administrators and/or any related third- party personnel.

In one or more embodiments, the AV safety driver training system 406 is in communication with an AV 102 for selectively controlling the AV 102 and/or for sending/receiving data (e.g., AV subsystem data) between the AVs 102 and the AV safety driver training system 406 via the network 702. The data associated with the AVs 102 includes, for example, vehicle operation fault data, driver response data, driver input values associated with one or more AV subsystems 112, AV health data (e.g., operational status of one or more AV subsystems 112), sensor data, real-time travel data, event data, process data, operational data, location data, and/or other data associated with the AVs 102. In various embodiments, one or more portions of said data associated the AVs 102 can be comprised in the AV safety driver training data 410. Additionally or alternatively, the data associated with the AVs 102 may include historical vehicle operation fault data, historical driver response data, historical driver input values associated with one or more AV subsystems 112, historical AV health data, historical sensor data, historical travel data, historical event data, historical process data, historical operational data, historical fault data, historical mission data, historical location data, and/or other historical data associated with the AVs 102. In various embodiments, one or more portions of said historical data associated the AVs 102 can be comprised in the AV safety driver training data 410.

In one or more embodiments, the AV safety driver training system 406 aggregates the data associated with the AVs 102. For instance, in one or more embodiments, the AV safety driver training system 406 aggregates the data associated with the AVs 102 into an AV safety driver training database 704. The AV safety driver training database 704 may be a cache memory (e.g., a database structure) that dynamically stores the data associated with the AVs 102.

In one or more embodiments, the AV safety driver training system 406 repeatedly updates data of the AV safety driver training database 704 based on the data provided by the AVs 102 during the one or more intervals of time associated with a particular AV safety driver training session. For instance, in one or more embodiments, the AV safety driver training system 406 stores new AV safety driver training data 410 and/or modified data associated with the progress of one or more AV safety driver training sessions associated with an AV 102. In one or more embodiments, the AV safety driver training system 406 formats one or more portions of the data associated with the AVs 102. For instance, in one or more embodiments, the AV safety driver training system 406 provides a formatted version of the data associated with the AVs 102 to the AV safety driver training database 704.

In various embodiments, the AV safety driver training system 406 is configured to generate and/or inject (e.g., initiate execution of) one or more vehicle operation faults and/or one or more compound vehicle operation faults associated with the one or more respective components of the control subsystem 114, vehicle drive subsystem 122, vehicle sensor subsystem 124, and/or vehicle control subsystem 128 associated with an AV 102. In various contexts, the one or more vehicle operation faults can cause one or more components associated with the one or more respective components of the control subsystem 114, vehicle drive subsystem 122, vehicle sensor subsystem 124, and/or vehicle control subsystem 128 to automatically engage, disengage, malfunction, and/or otherwise operate in a manner that is unexpected (e.g., dangerous, off-nominal) while the AV 102 is operating in an autonomous driving mode. For example, the one or more vehicle operation faults can include, but are not limited to, at least one of one or more steering faults, acceleration faults, deceleration faults, braking faults, stopping procedure faults, and/or vehicle trailer faults that cause one or more respective AV subsystems (e.g., a vehicle drive subsystem and/or a vehicle control subsystem) to automatically engage, disengage, malfunction, and/or otherwise operate in a manner that is unexpected.

As described herein, the one or more vehicle operation faults can be assigned a particular fault intensity value, where a fault intensity value is a relative input control value applied to the one or more AV subsystems 112 associated with an AV 102 operating in an autonomous driving mode. For example, a vehicle operation fault configured as a braking fault may be assigned a fault intensity value of a predefined percentage of the total braking capacity, such as 5%, 25%, 50%, 100% and/or the like, where the fault intensity value (e.g., 25%) is a relative input control value applied to via the brake unit 128b of an AV 102. For example, in the case of a braking fault injection with a fault intensity value of 25%, the AV safety driver training system 406 would cause the vehicle control subsystem 128 of the AV 102 to automatically apply the brakes of the brake unit 128b at 25% of the total braking capacity of the brake unit 128b. Similarly, a vehicle operation fault configured as a steering fault (e.g., a left drift or a right drift) may be assigned a fault intensity of an angular value related angular movement of the steering wheel, such as 8°, 20°, 30°, 90°, 120°, 180°, and/or the like, where the fault intensity value (e.g., 30°) is a relative input control value applied to the steering system (e.g., steering system 128d) of an AV 102. For example, in the case of a left drift steering fault injection with a fault intensity value of 30°, the AV safety driver training system 406 would cause the steering system 128d of the AV 102 to automatically turn 30° to the left from the current track of the AV 102.

In various embodiments, the AV safety driver training system 406 can receive selection of (e.g., by way of a user computing device 404) one or more compound vehicle operation faults. A compound vehicle operation fault can be understood as a combination of vehicle operation faults that affect two or more AV subsystems 112 simultaneously or sequentially. As a non-limiting example, the AV safety driver training system 406 can inject a compound vehicle operation fault into an AV 102 operating in an autonomous driving mode where the compound vehicle operation fault is configured to cause the AV 102 to simultaneously accelerate (e.g., inject an acceleration fault with a fault intensity value of 50% of the maximum acceleration capacity of the AV 102) and drift to the right (e.g., inject a right-steering fault with a fault intensity value of 20°).

Furthermore, in some embodiments, a vehicle operation fault and/or a compound vehicle operation fault can be injected during one or more predefined autonomous driving procedures associated with a particular AV 102. As a non-limiting example, a particular AV may be configured to execute a predefined, automatic stopping procedure if a stop sign, red traffic light, railroad crossing sign and/or the like is detected by one or more sensors 126 associated with the AV 102. In this regard, during a particular safety driver training session, a compound fault can be injected into an AV 102 that is performing a predefined, automatic stopping procedure. For example, as the AV 102 is executing a predefined, automatic stopping procedure (e.g., slowing down ahead of an upcoming stop sign), the AV safety driver training system 406 may inject a compound fault that causes the AV to accelerate (e.g., an acceleration fault with a fault intensity value of 100%) and drift to the left (e.g., a steering fault with a fault intensity value of 90°). The AV safety driver training system 406 can then determine one or more driver responses executed by the safety driver in response to the compound vehicle operation fault injected during the predefined, automatic stopping procedure being executed by the AV 102.

In various contexts, the AV safety driver training system 406 can be configured to assign a predefined fault injection duration to a respective vehicle operation fault and/or a compound vehicle operation fault. For example, a vehicle operation fault configured as an acceleration fault with a fault intensity value of 100% can be configured to have a fault injection duration of three seconds. In this particular scenario, once the acceleration fault is injected into the AV 102, a safety driver operating the AV 102 would have three seconds to respond correctly to the acceleration fault before the AV safety driver training system 406 removed (e.g., terminated, cancelled) the acceleration fault and the AV 102 returned to operating nominally in an autonomous driving mode.

As described herein, the AV safety driver training system 406 can be configured to determine one or more driver responses executed by a safety driver operating an AV 102 during a particular safety driver training session. The one or more driver responses can be understood to be one or more reactions of the safety driver to the one or more vehicle operation faults injected into the AV 102. In various contexts, the one or more driver responses can include, but are not limited to, at least one of a steering response, a braking response, an acceleration response, a deceleration response, and/or a gear shifting response. In this regard, the AV safety driver training system 406 is configured to interface with the AV 102 (e.g., via an in-vehicle control computer 103) to determine one or more driver input values associated with the one or more respective driver responses. The one or more driver input values are associated with one or more respective controls associated with the one or more vehicle systems associated with the AV 102.

As a non-limiting example, the AV safety driver training system 406 can be configured to determine a driver input value associated with one or more steering inputs executed by the safety driver (e.g., a degree or angular value and a directional value associated with the one or more respective steering inputs). As another non-limiting example, the AV safety driver training system 406 can be configured to determine a driver input value associated with one or more braking inputs executed by the safety driver (e.g., a braking system capacity percentage value associated with the one or more respective braking inputs).

In various embodiments, the AV safety driver training system 406 will automatically remove (e.g., terminate, cancel) the one or more vehicle operation faults upon detecting a driver response. Furthermore, in various embodiments, the AV safety driver training system 406 will automatically disengage the autonomous driving mode of the AV and switch the AV into a manual driving mode such that the safety driver assumes complete control of the AV 102 after executing the first driver response in reaction to the one or more vehicle operation faults. Furthermore, the AV safety driver training system 406 is configured to measure a reaction time of the safety driver. The reaction time is a measure of time it takes the safety driver to respond (e.g., execute one or more driver responses) to the one or more vehicle operation faults injected into the AV 102.

The AV safety driver training system 406 is also configured to generate, in response to injecting the one or more vehicle operation faults, one or more fault event tags. A fault event tag is an electronically managed data object associated with a particular vehicle operation fault or a particular compound vehicle operation fault. The one or more fault event tags comprise one or more portions of data related to at least one of a fault event tag identifier, the one or more vehicle operation faults, the one or more driver responses executed by the safety driver, the one or more reaction times associated with the safety driver, the one or more driver input values, and/or the one or more autonomous driving mode disengagement methods (e.g., a braking input, a steering input, etc.). In various embodiments, once a fault event tag is generated, the fault event tag is stored in an AV safety driver training database 704. Furthermore, various enterprise personnel (e.g., a safety driver and/or a safety driver trainer) can access the fault event tag (e.g., via the user computing device 404 associated with the AV safety driver training system 406) for reviewing, updating, commenting, and/or management purposes.

The AV safety driver training system 406 is also configured to generate an AV safety driver training session report. The AV safety driver training session report comprises at least one of one or more fault event tags, one or more portions of image data associated with the safety driver, one or more portions of video data associated with the safety driver, and/or one or more portions of audio data associated with the safety driver. An AV safety driver training session report is associated with a particular safety driver training session related to a particular safety driver and may be comprised of a plurality of digital files (e.g., text files, image files, video files, audio files, and/or the like) related to, e.g., capturing, the results of the particular safety driver training session. In various embodiments, one or more AV safety driver training session reports can be stored in an AV safety driver training database 704 and accessed, updated, deleted, and/or otherwise managed by the AV safety driver training system 406.

To capture image and/or audio data related to a particular safety driver training session, the cockpit of an AV 102 associated with an AV safety driver training system 406 can be outfitted with a DMK. In various embodiments, the DMK comprises a plurality of image capture devices

(e.g., video cameras) and/or audio recording devices (e.g., microphones) configured to capture image data related to the safety driver as the safety driver performs one or more safety driver training sessions and/or one or more real-world missions associated with a particular enterprise. The DMK can be configured to capture image data related to the hand placement, foot placement, head orientation and/or body posture of the safety driver.

Furthermore, the DMK can be configured to capture audio data (e.g., simultaneously with the image data) during the one or more safety driver training sessions and/or the one or more real-world missions. Moreover, the AV safety driver training system 406 can associate the image and/or audio data captured of a safety driver during a particular safety driver training session with one or more fault event tags by, for example, matching vehicle operation fault injection timestamp data with timestamp data related to the captured image and/or audio data. As such, the AV safety driver training system 406 can be configured to capture comprehensive data related to the driving patterns (e.g., reaction times, driver responses, body posture, manual driving hand placement, and/or the like) of a particular safety driver for use during one or more subsequent safety driver training sessions.

In various embodiments, AVs 102 associated with the AV safety driver training system 406 can be configured to operate on a specially designed safety track that is comprised of real-world roadway features such as signs, obstacles (e.g., other vehicles), markings, and/or the like. For example the safety track can comprise actual stop signs and/or stop lights such that preconfigured autonomous driving components (e.g., the AV subsystems 112) will cause the AV 102 to operate on the safety track in the same manner as the AV 102 would in the real-world environment upon encountering one or more roadway features. Non-limiting examples of real-world roadway features that can be incorporated on the safety track include, but are not limited to, stop signs, pedestrian crossing signs, railroad crossing signs, street signs, highway markers, traffic signals, lane markings, cross-walk markings, sidewalks, curbs, medians, and/or the like.

Furthermore, the routes associated with the safety track, as well as the corresponding roadway features, can be represented in a digital format and stored in a particular AV 102 (e.g., as map data 210 stored in an onboard a memory 210 and/or a map database 314). As such, various waypoints and/or or destinations associated with the safety track can be uploaded into the navigation system associated with the AV 102 such that the AV 102 can be directed to autonomously navigate the safety track during a safety driver training session.

In some embodiments, an AV 102 associated with the AV safety driver training system 406 can be configured to prioritize map data over perception data (e.g., sensor data) for how the AV plans trajectories (e.g., by way of the planning module 320), navigation routes, and/or one or more predefined autonomous driving procedures (e.g., predefined autonomous deceleration procedures, stopping procedures, turning procedures, engine braking procedures and/or the like) on the safety track. Additionally or alternatively, an AV 102 associated with the AV safety driver training system 406 can be configured to employ a combination of AV perception data (e.g., sensor data) and/or map data (e.g., map data 210) to plan one or more trajectories, navigation routes, and/or one or more predefined autonomous driving procedures.

In one or more embodiments, the user computing device 404 is in communication with the AV safety driver training system 406 via the network 702. In one or more embodiments, the user computing device 404 is a mobile computing device, a smartphone, a tablet computer, a mobile computer, a desktop computer, a laptop computer, a workstation computer, a wearable device, a virtual reality device, an augmented reality device, or another type of computing device located remote from the AV safety driver training system 406. In an embodiment, the user computing device 404 transmits the request 520 to the AV safety driver training system 406 via the network 702. In another embodiment, the AV safety driver training system 406 transmits the AV safety driver training data 410 to the user computing device 404, such as the fault injection system 408 of the user computing device 404, via the network 702.

In one or more embodiments, the AV safety driver training data 410 includes one or more visual elements for the visual display 604 of the user computing device 404 that renders the interactive safety driver training interface 402 based on a respective user interface configuration. In certain embodiments, the visual display 604 of the user computing device 404 displays one or more graphical elements associated with the AV safety driver training data 410. In certain embodiments, the visual display 604 of the user computing device 404 presents one or more interactive display elements associated with the AV safety driver training data 410. In another example, in one or more embodiments, the AV safety driver training data 410 includes one or notifications associated with the AV safety driver training data 410. In one or more embodiments, the AV safety driver training data 410 allows a user associated with the user computing device 404 to make decisions and/or perform one or more actions with respect to the AV 102.

In various embodiments, the interactive safety driver training interface 402 rendered via the visual display 604 of the user computing device 404 allows filtering of the AV safety driver training data and/or related interactive display elements. Additionally, one or more actions performed with respect to interactive display elements of the interactive safety driver training interface 402 of the user computing device 404 can initiate one or more actions with respect to the AV safety driver training system 406. For example, one or more actions performed with respect to interactive display elements of the interactive safety driver training interface 402 of the user computing device 404 can initiate an update to data stored in the AV safety driver training database 704 and/or an enterprise AV safety driver training protocol management portion of the AV safety driver training system 406.

### EXAMPLE PROCESSES OF THE PRESENT DISCLOSURE

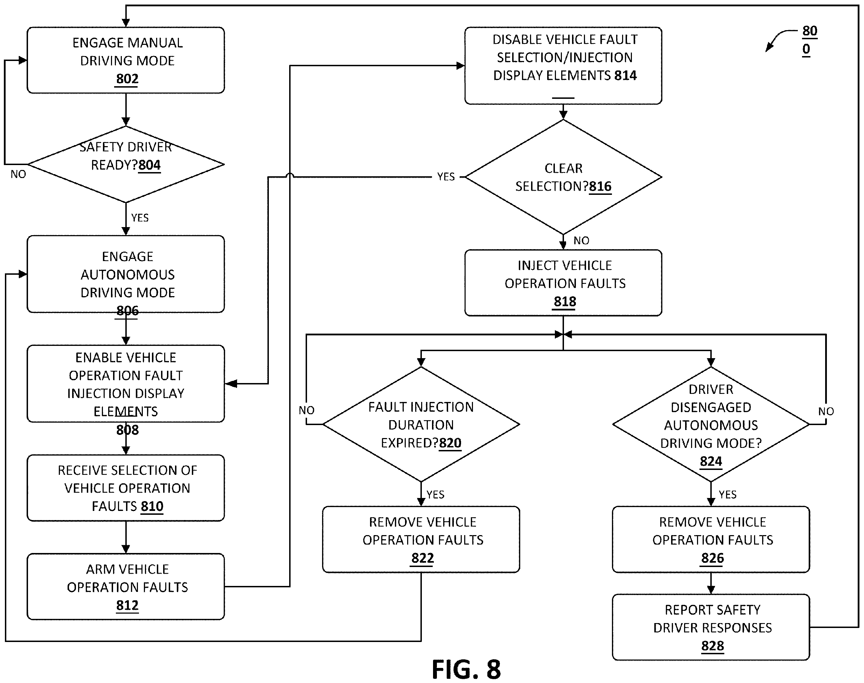

FIG. 8 is a process flow diagram of an example process 800 for executing a plurality of autonomous vehicle (AV) safety driver training operations in accordance with some embodiments discussed herein. In some embodiments, the process 800 is embodied by computer program code stored on a non-transitory computer-readable storage medium of a computer program product configured for execution to perform the process 800 as depicted and described.

Additionally or alternatively, in some embodiments, the process 800 is performed by one or more computing devices, such as the fault injection system 408 alone or in communication with one or more other component(s), device(s) (e.g., user computing device 404), and/or system(s) (e.g., AV safety driver training system 406). In this regard, in some such embodiments, the fault injection system 408 is specially configured by computer-coded instructions (e.g., computer program instructions) stored thereon, for example in the memory 512 and/or another component depicted and/or described herein and/or otherwise accessible to the fault injection system 408, for performing the operations as depicted and described. In some embodiments, the fault injection system 408 is in communication with one or more external apparatus(es), system(s), device(s), and/or the like, to perform one or more of the operations as depicted and described. For example, the fault injection system 408 in some embodiments is in communication with one or more system(s) integrated with, or embodying, an autonomous vehicle network platform (e.g., fault injection system 408 embodied by an autonomous vehicle network platform and integrated with the AV safety driver training system 406). For purposes of simplifying the description, the process 800 is described as performed by and from the perspective of the fault injection system 408 of the user computing device 404.

The process 800 begins at operation 802. At operation 802, the fault injection system 408 includes means, such as the communication component 504, the AV safety driver training component 506, the user interface component 508, the processor 510, and/or the memory 512, or any combination thereof, that engages a manual driving mode of an AV 102 associated with an AV safety driver training system 406. In this regard, the AV 102 is also associated with a safety driver participating in an AV safety driver training session. When the AV 102 is configured in the manual driving mode, the safety driver has complete control of one or more vehicle systems (e.g., AV subsystems 112) associated with the AV 102.

At operation 804, the fault injection system 408 includes means, such as the communication component 504, the AV safety driver training component 506, the user interface component 508, the processor 510, and/or the memory 512, or any combination thereof, that determines whether the safety driver is prepared to relinquish control of the AV 102 to the autonomous driving components associated with the AV subsystems 112, such as in an instance in which the AV 102 is presently operating in a safe and predictable manner and the safety driver is properly positioned within the AV. If it is determined that the safety driver is so prepared, the process 800 proceeds to operation 806. However, if it is determined that the safety driver is not ready to relinquish control of the AV 102, such as in an instance in which the safety driver is in the process of making a course adjustment, a speed adjustment or the like, the process 800 reverts to operation 802 (e.g., the AV 102 remains in the manual driving mode).

At operation 806, the fault injection system 408 includes means, such as the communication component 504, the AV safety driver training component 506, the user interface component 508, the processor 510, and/or the memory 512, or any combination thereof, that engages an autonomous driving mode of the AV 102. In various embodiments, the autonomous driving mode can be a fully-autonomous driving mode or a partially-autonomous driving mode. In various other embodiments, the AV safety driver training system 406 can configure whether the AV 102 engages a fully-autonomous driving mode or a partially-autonomous driving mode.

At operation 808, the fault injection system 408 includes means, such as the communication component 504, the AV safety driver training component 506, the user interface component 508, the processor 510, and/or the memory 512, or any combination thereof, that enables one or more interactive display elements associated with one or more respective vehicle operation fault injection configuration parameters. As described herein, in one or more embodiments, the interactive safety driver training interface 402 is configured to provide dynamic interaction with vehicle operation fault configuration parameters that are rendered as respective interactive display elements via the interactive safety driver training interface 402. An interactive display element is a portion of the interactive safety driver training interface 402 (e.g., a user-interactive electronic interface portion) that provides for interaction with respect to an end user of the user computing device 404. For example, in one or more embodiments, an interactive display element is an interactive display element that allows a user to provide feedback and/or to perform one or more actions with respect to the interactive safety driver training interface 402. Non-limiting examples of interactive display elements can include interactive buttons, sliders, hyperlinks, text input fields, graphs, charts, and/or tables. The AV safety driver training system 406 can enable or disable one or more interactive display elements associated with the interactive safety driver training interface 402 during an AV safety driver training session depending on a current status of the AV 102 and/or depending on whether one or more vehicle operation faults are currently being executed in the AV 102.

At operation 810, the fault injection system 408 includes means, such as the communication component 504, the AV safety driver training component 506, the user interface component 508, the processor 510, and/or the memory 512, or any combination thereof, that receives selection of one or more vehicle operation faults. For example, as described herein, one or more interactive display elements associated with one or more respective vehicle operation fault injection configuration parameters can be rendered for selection by the trainer on the interactive safety driver training interface 402.

At operation 812 and in response to selection by the trainer of one or more vehicle operation faults, the fault injection system 408 includes means, such as the communication component 504, the AV safety driver training component 506, the user interface component 508, the processor 510, and/or the memory 512, or any combination thereof, that arms one or more vehicle operation faults for injection into the AV 102. For example, the one or more vehicle operation faults that were generated by the selection of the one or more respective vehicle operation fault injection configuration parameters at operation 810 can be armed for injection by the AV safety driver training system 406, thereby preparing the AV 102 and the subsystems thereof for imposition of the one or more vehicle operation faults.

At operation 814, the fault injection system 408 includes means, such as the communication component 504, the AV safety driver training component 506, the user interface component 508, the processor 510, and/or the memory 512, or any combination thereof, that disables one or more interactive display elements associated with the one or more respective vehicle operation fault injection configuration parameters. For example, the interactive display elements can be disabled in response to the one or more vehicle operation faults being armed.

At operation 816, the fault injection system 408 includes means, such as the communication component 504, the AV safety driver training component 506, the user interface component 508, the processor 510, and/or the memory 512, or any combination thereof, that determines whether the selection of the one or more vehicle operation fault configuration parameters associated with the one or more vehicle operation faults should be cleared. For example, in some embodiments, an interactive button can be rendered via the interactive safety driver training interface 402 that is configured to clear a selection of previously selected vehicle operation fault configuration parameters upon actuation by the trainer. If the AV safety driver training system 406 determines that the selection of vehicle operation fault configuration parameters should be cleared, the process 800 clears the selection and reverts to operation 808, thereby re-enabling the interactive display elements associated with the one or more respective vehicle operation fault configuration parameters. If the AV safety driver training system 406 determines that the selection of vehicle operation fault configuration parameters should not be cleared, the process 800 proceeds to operation 818.

At operation 818, the fault injection system 408 includes means, such as the communication component 504, the AV safety driver training component 506, the user interface component 508, the processor 510, and/or the memory 512, or any combination thereof, that injects the one or more vehicle operation faults, thereby causing the one or more vehicle operation faults to occur. In some embodiments, the AV safety driver training system 406 injects the one or more vehicle operation faults in response to an interaction with an interactive display element rendered on the interactive safety driver training interface 402.

At operation 820, the fault injection system 408 includes means, such as the communication component 504, the AV safety driver training component 506, the user interface component 508, the processor 510, and/or the memory 512, or any combination thereof, that determines whether a predefined fault injection duration associated with the one or more vehicle operation faults has expired. As described herein, the AV safety driver training system 406 can assign a predefined fault injection duration to a respective vehicle operation fault and/or a compound vehicle operation fault. For example, a vehicle operation fault configured as an acceleration fault with a fault intensity value of 100% can be configured to have a fault injection duration of three seconds. In this scenario, once the acceleration fault is injected into the AV 102, a safety driver at the AV 102 would have three seconds to respond correctly to the acceleration fault before the AV safety driver training system 406 removes (e.g., terminated, cancelled) the acceleration fault and the AV 102 is returned to operating nominally in an autonomous driving mode. If the AV safety driver training system 406 determines that the predefined fault injection duration has not expired, the AV safety driver training system continues to monitor for expiration of the predefined fault injection duration. However, if the AV safety driver training system 406 determines that the predefined fault injection duration has expired, the process 800 proceeds to operation 822.

At operation 824, the fault injection system 408 includes means, such as the communication component 504, the AV safety driver training component 506, the user interface component 508, the processor 510, and/or the memory 512, or any combination thereof, that determines whether the driver has disengaged the autonomous driving mode of the AV 102. As described herein, in various embodiments, the AV safety driver training system 406 will automatically remove (e.g., terminate, cancel) the one or more vehicle operation faults upon detecting a safety driver response. Furthermore, in various embodiments, the AV safety driver training system 406 will automatically disengage the autonomous driving mode of the AV 102 and switch the AV 102 into a manual driving mode such that the safety driver assumes complete control of the AV 102 after executing the first driver response in reaction to the one or more vehicle operation faults. If the AV safety driver training system 406 determines that the driver has not disengaged the autonomous driving mode, the AV safety driver training system continues to monitor for disengagement of the autonomous driving mode. However, if the AV safety driver training system 406 determines that the safety driver has disengaged the autonomous driving mode of the AV 102, the process 800 proceeds to operation 826.

At operation 826, the fault injection system 408 includes means, such as the communication component 504, the AV safety driver training component 506, the user interface component 508, the processor 510, and/or the memory 512, or any combination thereof, that removes (e.g., terminates, cancels) the one or more vehicle operation faults currently being injected into the AV 102.

At operation 828, the fault injection system 408 includes means, such as the communication component 504, the AV safety driver training component 506, the user interface component 508, the processor 510, and/or the memory 512, or any combination thereof, that records and reports one or more driver responses executed in response to the one or more vehicle operation faults that were injected into the AV 102. As described herein, the AV safety driver training system 406 is also configured to generate an AV safety driver training session report. The AV safety driver training session report comprises at least one of one or more fault event tags, one or more portions of image data associated with the safety driver, one or more portions of video data associated with the safety driver, and/or one or more portions of audio data associated with the safety driver. An AV safety driver training session report is associated with a particular safety driver training session related to a particular safety driver and may be comprised of a plurality of digital files (e.g., text files, image files, video files, audio files, and/or the like) related to the results of the particular safety driver training session. In various embodiments, one or more AV safety driver training session reports can be stored in an AV safety driver training database 704 and accessed, updated, deleted, and/or otherwise managed by the AV safety driver training system 406. In various embodiments, after one or more driver responses are detected, the process 800 reverts to operation 802 and the AV safety driver training system 406 switches the AV 102 into a manual driving mode in which the safety driver assumes complete control of the AV subsystems 112.

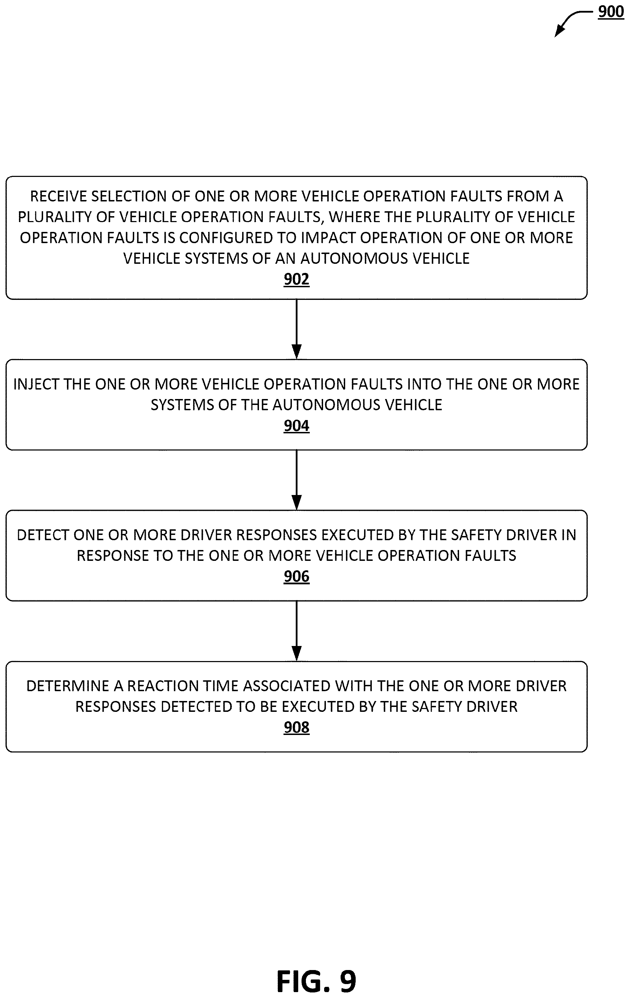

FIG. 9 is a process flow diagram of an example process 900 for executing a plurality of AV safety driver training operations in accordance with some embodiments discussed herein. In some embodiments, the process 900 is embodied by computer program code stored on a non-transitory computer-readable storage medium of a computer program product configured for execution to perform the process as depicted and described.

Additionally or alternatively, in some embodiments, the process 900 is performed by one or more computing devices, such as the fault injection system 408 alone or in communication with one or more other component(s), device(s) (e.g., user computing device 404), and/or system(s) (e.g., AV safety driver training system 406). In this regard, in some such embodiments, the fault injection system 408 is specially configured by computer-coded instructions (e.g., computer program instructions) stored thereon, for example in the memory 512 and/or another component depicted and/or described herein and/or otherwise accessible to the fault injection system 408, for performing the operations as depicted and described. In some embodiments, the fault injection system 408 is in communication with one or more external apparatus(es), system(s), device(s), and/or the like, to perform one or more of the operations as depicted and described. For example, the fault injection system 408 in some embodiments is in communication with one or more system(s) integrated with, or embodying, an autonomous vehicle network platform (e.g., fault injection system 408 embodied by an autonomous vehicle network platform and integrated with the AV safety driver training system 406). For purposes of simplifying the description, the process 900 is described as performed by and from the perspective of the fault injection system 408 of the user computing device 404.

The process 900 begins at operation 902. At operation 902, the fault injection system 408 includes means, such as the communication component 504, the AV safety driver training component 506, the user interface component 508, the processor 510, and/or the memory 512, or any combination thereof, that receives selection, such as from the trainer, of one or more vehicle operation faults from a plurality of vehicle operation faults. The plurality of vehicle operation faults are configured to impact operation of one or more vehicle subsystems associated with the AV.

At operation 904, the fault injection system 408 includes means, such as the communication component 504, the AV safety driver training component 506, the user interface component 508, the processor 510, and/or the memory 512, or any combination thereof, that injects the one or more vehicle operation faults into one or more components of the AV 102, thereby causing the AV 102 to respond in an manner that is unanticipated by the safety driver.

At operation 906, the fault injection system 408 includes means, such as the communication component 504, the AV safety driver training component 506, the user interface component 508, the processor 510, and/or the memory 512, or any combination thereof, that detects one or more driver responses executed by the safety driver. The one or more driver responses are characterized by a reaction of the safety driver to the one or more vehicle operation faults.

At operation 908, the fault injection system 408 includes means, such as the communication component 504, the AV safety driver training component 506, the user interface component 508, the processor 510, and/or the memory 512, or any combination thereof, that determines a reaction time associated with the one or more driver responses detected to be executed by the safety driver. The reaction time is a measure of time it takes the safety driver to respond to the one or more vehicle operation faults injected into the one or more vehicle systems of the autonomous vehicle.

### OPERATIONAL EXAMPLES OF THE PRESENT DISCLOSURE

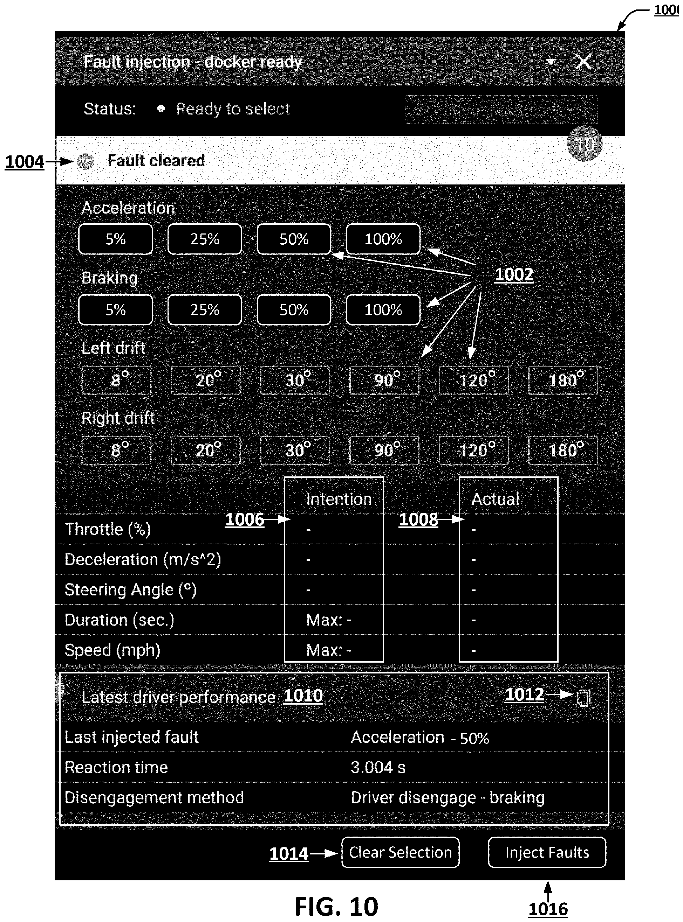

FIG. 10 is an operational example of an interactive safety driver training interface 1000 in accordance with some embodiments discussed herein. In one or more embodiments, a user computing device 404 can generate and/or render one or more interactive display elements associated with various portions of AV safety driver training data 410 that has been compiled by the AV safety driver training system 406 related to a particular AV safety driver training session. Non-limiting examples of interactive display elements can include interactive buttons, sliders, hyperlinks, text input fields, graphs, charts, and/or tables.

For example, the user computing device 404, in conjunction with the AV safety driver training system 406 can generate one or more interactive display elements such as the interactive display element(s) 1002 rendered as interactive buttons/selectors associated with various vehicle operation fault injection configuration parameters. Selection of one or more of the interactive display elements 1103 by the trainer begins the process described above of injecting the corresponding vehicle operation fault. For example, selection of the interactive display elements 1103 associated with a 25% acceleration and a right drift of 30° will begin the process of injecting the compound fault. The interactive safety driver training interface 402 of FIG. 10 also includes a display element 1004 configured as a notification banner associated with the current status of a particular vehicle operation fault, such as by indicating if a fault is injected or has been cleared.

The interactive safety driver training interface 402 of this example embodiment also includes a display element 1006 rendered as a grouping of text fields configured to display one or more portions of AV safety driver training data 410 associated with an intended (e.g., preconfigured) set of fault intensity values related to a particular vehicle operation fault and a display element 1008 rendered as a grouping of text fields configured to display actual driver input values associated with one or more safety driver responses that were executed in response to the particular vehicle operation fault. Thus, in response to an injected fault, display element 1006 would indicate the anticipated value of several vehicle parameters, e.g., throttle, deceleration, etc., and display element 1008 would indicate the actual value of those same vehicle parameters with any difference being attributable to the safety driver responses to the injected fault.

The interactive safety driver training interface 402 of this example embodiment also includes a section 1010 summarizing the performance of the safety driver to the latest injected fault including the type of injected fault, the reaction time of the safety driver to address the injected fault and the disengagement method. In this example embodiment, the interactive safety driver training interface 402 may also include an interactive display element 1012 rendered as an interactive icon/button configured to generate, in response to actuation, a request 520 to manually generate a fault event tag associated with a particular vehicle operation fault to capture information regarding the injected fault and the response of the safety driver. The interactive safety driver training interface 402 of this example embodiment also includes an interactive display element 1014 rendered as an interactive button configured to clear a selection of any vehicle operation fault and an interactive display element 1016 rendered as an interactive button configured to inject one or more vehicle operations faults based on one or more fault injection configuration parameters that were previously selected (e.g., via interactive display elements 1002).

The various interactive display elements can be configured on the interactive safety driver training interface 402 such that an interaction with the interactive display elements by the trainer causes the user computing device 404 to execute various actions. These actions may include, but are not limited to, reconfiguring the interactive safety driver training interface 402 and/or transmitting various signals to the AV safety driver training system 406 causing the AV safety driver training system 406 to execute various functions (e.g., initiate one or more vehicle operation fault injections).

As described herein, various portions of AV safety driver training data 410 associated with a particular AV 102 can be rendered as one or more respective interactive display elements on the interactive safety driver training interface 402. In various contexts, the AV safety driver training data 410 rendered on the interactive safety driver training interface 402 can be real-time data associated with a current AV safety driver training session such as, for example, real-time driver input values associated with one or more AV subsystems 112 related to one or more driver responses to a particular vehicle operation fault. In some embodiments, the interactive display element 1008 associated may be configured to render real-time driver input values related to one or more driver responses executed in response to a particular vehicle operation fault injected into the AV 102.

In various other contexts, the AV safety driver training data 410 rendered on the interactive safety driver training interface 402 can be historical data such as, for example, data related to one or more fault event tags and/or data related to one or more AV safety driver training session reports. In one or more embodiments, the display element 1010 can be configured to render historical AV safety driver training data 410 related to a latest driver performance. For example, as shown in FIG. 10, the display element 1010 has been configured to display AV safety driver training data 410 comprising a last injected vehicle operation fault type and corresponding fault intensity value, a driver reaction time associated with a safety driver that responded to the last injected vehicle operation fault, as well as an autonomous driving mode disengagement method executed by the safety driver.

In one or more embodiments, an interaction with one or more interactive display elements rendered on the interactive safety driver training interface 402 can be employed to initialize one or more processes and/or actions associated with a particular AV 102. As a non-limiting example, an interaction with the interactive display element(s) 1002 followed by selection of the interactive display element 1016 to inject the selected faults can cause the AV safety driver training system 406 to generate one or more vehicle operation faults based on the on one or more fault injection configuration parameters associated with the interactive display element(s) 1002 that have been selected.

As another non-limiting example, an interaction with the interactive display element 1012 can cause the AV safety driver training system 406 to generate a fault event tag associated with the AV safety driver training data 410 currently rendered by the display element 1010. Once generated, the fault event tag can be stored in one or more data stores associated with the AV safety driver training system 406 (e.g., the AV safety driver training database 704).

As yet another non-limiting example, an interaction with the interactive display element 1014 can cause the AV safety driver training system 406 to clear a selection of one or more fault injection configuration parameters that were previously selected (e.g., via interactive display elements 1002).

In general, methods, apparatus, systems, computing devices, computing entities, and/or the like are therefore provided for allowing a trainer to controllably inject faults to one or more components of an AV 102 and to monitor the response of a safety driver to the faults. The trainer may be onboard the AV 102 with the safety driver with the interactive safety driver training interface 402 provided by a user computing device 404 carried by the trainer or otherwise accessible to the trainer within the AV 102. Alternatively, the trainer may be remote from the AV 102, but in communication therewith. In either instance, if the safety driver fails to respond properly to the injected fault, the trainer has the option of clearing the fault and either allowing the AV 102 to reassume autonomous driving operations or to allow for the AV to be manually driven, either by the trainer or the safety driver, thereby ensuring the continued safety of the AV 102 and the participants. By reviewing the response of the safety driver, such as my be recorded by a fault event tag, the trainer can coach the safety driver as to how to better respond to the various faults and the process can be repeated any number of times until the safety driver has mastered the response to the various types of faults and is prepared to serve as a safety driver for an AV in normal operating conditions..

FIG(s). 8 and 9 illustrate operations of an apparatus, computer-implemented method, and computer program product according to some example embodiments. It will be understood that each operation of the flowcharts or diagrams, and combinations of operations in the flowcharts or diagrams, may be implemented by various means, such as hardware and/or a computer program product comprising one or more computer-readable mediums having computer readable program instructions stored thereon. For example, one or more of the procedures described herein may be embodied by computer program instructions of a computer program product. In this regard, the computer program product(s) which embody the procedures described herein may comprise one or more memory devices storing instructions executable by a processor. In some example embodiments, the computer program instructions of the computer program product(s) which embody the procedures described above may be stored by memory devices of a plurality of computing devices.

As will be appreciated, any such computer program product may be loaded onto a computer or other programmable apparatus to produce a machine, such that the computer program product including the instructions which execute on the computer or other programmable apparatus creates means for implementing the functions specified in the flowchart block(s). Further, the computer program product may comprise one or more computer-readable memories on which the computer program instructions may be stored such that the one or more computer-readable memories can direct a computer or other programmable apparatus to function in a particular manner, such that the computer program product may comprise an article of manufacture which implements the function specified in the flowchart block(s). The computer program instructions of one or more computer program products may also be loaded onto a computer or other programmable apparatus (for example, in-vehicle control computer 103, user computing device 404, fault injection system 408 and/or other apparatus) to cause a series of operations to be performed on the computer or other programmable apparatus to produce a computer-implemented process such that the instructions which execute on the computer or other programmable apparatus implement the functions specified in the flowchart block(s).

Accordingly, blocks of the flowcharts support combinations of means for performing the specified functions and combinations of operations for performing the specified functions. It will also be understood that one or more blocks of the flowcharts, and combinations of blocks in the flowcharts, can be implemented by special purpose hardware-based computer systems which perform the specified functions, or combinations of special purpose hardware and computer instructions.

Clause 1. An apparatus comprising at least one processor and at least one non-transitory memory including computer program code instructions, the computer program code instructions configured to, when executed, cause the apparatus to:
receive a selection of one or more vehicle operation faults from a plurality of vehicle operation faults, wherein the plurality of vehicle operation faults is configured to impact an operation of one or more vehicle systems of an autonomous vehicle operating in an autonomous driving mode;
inject the one or more vehicle operation faults to the one or more vehicle systems of the autonomous vehicle;
detect one or more driver responses executed by a safety driver in response to the one or more vehicle operation faults; and
determine a reaction time associated with the one or more driver responses detected to be executed by the safety driver, wherein the reaction time is a measure of time it takes the safety driver to respond to the one or more vehicle operation faults injected into the one or more vehicle systems of the autonomous vehicle.

Clause 2. The apparatus of Clause 1, wherein the plurality of vehicle operation faults comprises at least one of one or more steering faults, acceleration faults, deceleration faults, braking faults, stopping procedure faults, or vehicle trailer faults.

Clause 3. The apparatus of any of Clauses 1 or 2, wherein each vehicle operation fault of the plurality of vehicle operation faults is associated with a predefined fault injection duration.

Clause 4. The apparatus of any of Clauses 1 to 3, wherein the computer program code instructions configured to detect the one or more driver responses executed by the safety driver further cause the apparatus to:
determine one or more driver input values associated with the one or more respective driver responses to the one or more vehicle operation faults, wherein the one or more driver input values are associated with one or more respective controls associated with the one or more vehicle systems of the autonomous vehicle.

Clause 5. The apparatus of any of Clauses 1 to 4, wherein the one or more driver responses comprise at least one of a steering response, a braking response, an acceleration response, a deceleration response, or a gear shifting response.

Clause 6. The apparatus of any of Clauses 1 to 5, wherein at least one of the one or more driver responses causes the autonomous vehicle to disengage from the autonomous driving mode and switch to a manual driving mode.

Clause 7. The apparatus of any of Clauses 1 to 6, wherein the computer program code instructions further cause the apparatus to:
generate, in response to injecting the one or more vehicle operation faults, one or more fault event tags, wherein the one or more fault event tags comprise one or more portions of data related to at least one of a fault event tag identifier, vehicle operation fault injection timestamp data, the one or more vehicle operation faults, the one or more driver responses executed by the safety driver in response to the one or more vehicle operation faults, one or more reaction times associated with the safety driver, one or more driver input values, or one or more autonomous driving mode disengagement methods.

Clause 8. The apparatus of any of Clauses 1 to 7, wherein the computer program code instructions further cause the apparatus to:
generate an AV safety driver training session report, wherein the AV safety driver training session report comprises at least one of one or more fault event tags, one or more portions of image data associated with the safety driver, one or more portions of video data associated with the safety driver, or one or more portions of audio data associated with the safety driver, and wherein the safety driver training session report is associated with a respective AV safety driver training session of the safety driver.

Clause 9. A computer-implemented method, the computer-implemented method comprising:
receiving a selection of one or more vehicle operation faults from a plurality of vehicle operation faults, wherein the plurality of vehicle operation faults is configured to impact an operation of one or more vehicle systems of an autonomous vehicle operating in an autonomous driving mode;
injecting the one or more vehicle operation faults to the one or more vehicle systems of the autonomous vehicle;
detecting one or more driver responses executed by a safety driver in response to the one or more vehicle operation faults; and
determining a reaction time associated with the one or more driver responses detected to be executed by the safety driver, wherein the reaction time is a measure of time it takes the safety driver to respond to the one or more vehicle operation faults injected into the one or more vehicle systems of the autonomous vehicle.

Clause 10. The computer-implemented method of Clause 9, wherein the plurality of vehicle operation faults comprises at least one of one or more steering faults, acceleration faults, deceleration faults, braking faults, stopping procedure faults, or vehicle trailer faults.

Clause 11. The computer-implemented method of any of Clauses 9 or 10, wherein each vehicle operation fault of the plurality of vehicle operation faults is associated with a predefined fault injection duration.

Clause 12. The computer-implemented method of any of Clauses 9 to 11, wherein detecting the one or more driver responses executed by the safety driver comprises:
determining one or more driver input values associated with the one or more respective driver responses to the one or more vehicle operation faults, wherein the one or more driver input values are associated with one or more respective controls associated with the one or more vehicle systems of the autonomous vehicle.

Clause 13. The computer-implemented method of any of Clauses 9 to 12, wherein the one or more driver responses comprise at least one of a steering response, a braking response, an acceleration response, a deceleration response, or a gear shifting response.

Clause 14. The computer-implemented method of any of Clauses 9 to 13, wherein at least one of the one or more driver responses causes the autonomous vehicle to disengage from the autonomous driving mode and switch to a manual driving mode.

Clause 15. The computer-implemented method of any of Clauses 9 to 14, further comprising:
generating, in response to injecting the one or more vehicle operation faults, one or more fault event tags, wherein the one or more fault event tags comprise one or more portions of data related to at least one of a fault event tag identifier, vehicle operation fault injection timestamp data, the one or more vehicle operation faults, the one or more driver responses executed by the safety driver in response to the one or more vehicle operation faults, one or more reaction times associated with the safety driver, one or more driver input values, or one or more autonomous driving mode disengagement methods.

Clause 16. The computer-implemented method of any of Clauses 9 to 15, further comprising:
generating an AV safety driver training session report, wherein the AV safety driver training session report comprises at least one of one or more fault event tags, one or more portions of image data associated with the safety driver, one or more portions of video data associated with the safety driver, or one or more portions of audio data associated with the safety driver, and wherein the safety driver training session report is associated with a respective AV safety driver training session of the safety driver.

Clause 17. A computer program product comprising at least one non-transitory computer-readable storage medium having computer-executable program code instructions stored therein, the computer-executable program code instructions comprising program code instructions configured to:
receive a selection of one or more vehicle operation faults from a plurality of vehicle operation faults, wherein the plurality of vehicle operation faults is configured to impact an operation of one or more vehicle systems of an autonomous vehicle operating in an autonomous driving mode;
inject the one or more vehicle operation faults to the one or more vehicle systems of the autonomous vehicle;
detect one or more driver responses executed by a safety driver in response to the one or more vehicle operation faults; and
determine a reaction time associated with the one or more driver responses detected to be executed by the safety driver, wherein the reaction time is a measure of time it takes the safety driver to respond to the one or more vehicle operation faults injected into the one or more vehicle systems of the autonomous vehicle.

Clause 18. The computer program product of Clause 17, wherein the plurality of vehicle operation faults comprises at least one of one or more steering faults, acceleration faults, deceleration faults, braking faults, stopping procedure faults, or vehicle trailer faults.

Clause 19. The computer program product of any of Clauses 17 or 18, wherein each vehicle operation fault of the plurality of vehicle operation faults is associated with a predefined fault injection duration.

Clause 20. The computer program product of any of Clauses 17 to 19, wherein the program code instructions configured to detect the one or more driver responses executed by the safety driver comprise program code instructions configured to:
determine one or more driver input values associated with the one or more respective driver responses to the one or more vehicle operation faults, wherein the one or more driver input values are associated with one or more respective controls associated with the one or more vehicle systems of the autonomous vehicle.

Clause 21. The computer program product of any of Clauses 17 to 20, wherein the one or more driver responses comprise at least one of a steering response, a braking response, an acceleration response, a deceleration response, or a gear shifting response.

Clause 22. The computer program product of any of Clauses 17 to 21, wherein at least one of the one or more driver responses causes the autonomous vehicle to disengage from the autonomous driving mode and switch to a manual driving mode.

Clause 23. The computer program product of any of Clauses 17 to 22, wherein the computer-executable program code instructions further comprise program code instructions configured to:
generate, in response to injecting the one or more vehicle operation faults, one or more fault event tags, wherein the one or more fault event tags comprise one or more portions of data related to at least one of a fault event tag identifier, vehicle operation fault injection timestamp data, the one or more vehicle operation faults, the one or more driver responses executed by the safety driver in response to the one or more vehicle operation faults, one or more reaction times associated with the safety driver, one or more driver input values, or one or more autonomous driving mode disengagement methods.

Clause 24. The apparatus of any of Clauses 22 to 23, wherein the computer-executable program code instructions further comprise program code instructions configured to:
generate an AV safety driver training session report, wherein the AV safety driver training session report comprises at least one of one or more fault event tags, one or more portions of image data associated with the safety driver, one or more portions of video data associated with the safety driver, or one or more portions of audio data associated with the safety driver, and wherein the safety driver training session report is associated with a respective AV safety driver training session of the safety driver.

Many modifications and other embodiments of the inventions set forth herein will come to mind to one skilled in the art to which these inventions pertain having the benefit of the teachings presented in the foregoing descriptions and the associated drawings. Therefore, it is to be understood that the inventions are not to be limited to the specific embodiments disclosed and that modifications and other embodiments are intended to be included within the scope of the appended claims. Moreover, although the foregoing descriptions and the associated drawings describe example embodiments in the context of certain example combinations of elements and/or functions, it should be appreciated that different combinations of elements and/or functions may be provided by alternative embodiments without departing from the scope of the appended claims. In this regard, for example, different combinations of elements and/or functions than those explicitly described above are also contemplated as may be set forth in some of the appended claims. Although specific terms are employed herein, they are used in a generic and descriptive sense only and not for purposes of limitation.

## Claims

1. An apparatus comprising at least one processor and at least one non-transitory memory including computer program code instructions, the computer program code instructions configured to, when executed, cause the apparatus to:
receive a selection of one or more vehicle operation faults from a plurality of vehicle operation faults, wherein the plurality of vehicle operation faults is configured to impact an operation of one or more vehicle systems of an autonomous vehicle operating in an autonomous driving mode;
inject the one or more vehicle operation faults to the one or more vehicle systems of the autonomous vehicle;
detect one or more driver responses executed by a safety driver in response to the one or more vehicle operation faults; and
determine a reaction time associated with the one or more driver responses detected to be executed by the safety driver, wherein the reaction time is a measure of time it takes the safety driver to respond to the one or more vehicle operation faults injected into the one or more vehicle systems of the autonomous vehicle.

2. The apparatus of Claim 1, wherein the plurality of vehicle operation faults comprises at least one of one or more steering faults, acceleration faults, deceleration faults, braking faults, stopping procedure faults, or vehicle trailer faults.

3. The apparatus of any of Claims 1 or 2, wherein each vehicle operation fault of the plurality of vehicle operation faults is associated with a predefined fault injection duration.

4. The apparatus of any of Claims 1 to 3, wherein the computer program code instructions configured to detect the one or more driver responses executed by the safety driver further cause the apparatus to:
determine one or more driver input values associated with the one or more respective driver responses to the one or more vehicle operation faults, wherein the one or more driver input values are associated with one or more respective controls associated with the one or more vehicle systems of the autonomous vehicle.

5. The apparatus of any of Claims 1 to 4, wherein the one or more driver responses comprise at least one of a steering response, a braking response, an acceleration response, a deceleration response, or a gear shifting response, or/and optionally wherein at least one of the one or more driver responses causes the autonomous vehicle to disengage from the autonomous driving mode and switch to a manual driving mode.

6. The apparatus of any of Claims 1 to 5, wherein the computer program code instructions further cause the apparatus to:
generate, in response to injecting the one or more vehicle operation faults, one or more fault event tags, wherein the one or more fault event tags comprise one or more portions of data related to at least one of a fault event tag identifier, vehicle operation fault injection timestamp data, the one or more vehicle operation faults, the one or more driver responses executed by the safety driver in response to the one or more vehicle operation faults, one or more reaction times associated with the safety driver, one or more driver input values, or one or more autonomous driving mode disengagement methods.

7. The apparatus of any of Claims 1 to 6, wherein the computer program code instructions further cause the apparatus to:
generate an AV safety driver training session report, wherein the AV safety driver training session report comprises at least one of one or more fault event tags, one or more portions of image data associated with the safety driver, one or more portions of video data associated with the safety driver, or one or more portions of audio data associated with the safety driver, and wherein the safety driver training session report is associated with a respective AV safety driver training session of the safety driver.

8. A computer-implemented method, the computer-implemented method comprising:
receiving a selection of one or more vehicle operation faults from a plurality of vehicle operation faults, wherein the plurality of vehicle operation faults is configured to impact an operation of one or more vehicle systems of an autonomous vehicle operating in an autonomous driving mode;
injecting the one or more vehicle operation faults to the one or more vehicle systems of the autonomous vehicle;
detecting one or more driver responses executed by a safety driver in response to the one or more vehicle operation faults; and
determining a reaction time associated with the one or more driver responses detected to be executed by the safety driver, wherein the reaction time is a measure of time it takes the safety driver to respond to the one or more vehicle operation faults injected into the one or more vehicle systems of the autonomous vehicle.

9. The computer-implemented method of Claim 8, wherein the plurality of vehicle operation faults comprises at least one of one or more steering faults, acceleration faults, deceleration faults, braking faults, stopping procedure faults, or vehicle trailer faults.

10. The computer-implemented method of any of Claims 8 or 9, wherein each vehicle operation fault of the plurality of vehicle operation faults is associated with a predefined fault injection duration.

11. The computer-implemented method of any of Claims 8 to 10, wherein detecting the one or more driver responses executed by the safety driver comprises:
determining one or more driver input values associated with the one or more respective driver responses to the one or more vehicle operation faults, wherein the one or more driver input values are associated with one or more respective controls associated with the one or more vehicle systems of the autonomous vehicle.

12. The computer-implemented method of any of Claims 8 to 11, wherein the one or more driver responses comprise at least one of a steering response, a braking response, an acceleration response, a deceleration response, or a gear shifting response, or/and optionally wherein at least one of the one or more driver responses causes the autonomous vehicle to disengage from the autonomous driving mode and switch to a manual driving mode.

13. The computer-implemented method of any of Claims 8 to 12, further comprising:
generating, in response to injecting the one or more vehicle operation faults, one or more fault event tags, wherein the one or more fault event tags comprise one or more portions of data related to at least one of a fault event tag identifier, vehicle operation fault injection timestamp data, the one or more vehicle operation faults, the one or more driver responses executed by the safety driver in response to the one or more vehicle operation faults, one or more reaction times associated with the safety driver, one or more driver input values, or one or more autonomous driving mode disengagement methods.

14. The computer-implemented method of any of Claims 8 to 13, further comprising: generating an AV safety driver training session report, wherein the AV safety driver training session report comprises at least one of one or more fault event tags, one or more portions of image data associated with the safety driver, one or more portions of video data associated with the safety driver, or one or more portions of audio data associated with the safety driver, and wherein the safety driver training session report is associated with a respective AV safety driver training session of the safety driver.

15. A computer program product comprising at least one non-transitory computer-readable storage medium having computer-executable program code instructions stored therein, the computer-executable program code instructions comprising program code instructions configured to perform the method of any of claims 8 to 14.
